# EUROPEAN PATENT SPECIFICATION

(11) **EP 1 720 993 B1**
(45) Date of publication and mention of the grant of the patent: **16.10.2013**
(21) Application number: 05710272.5
(22) Date of filing: 09.02.2005
(51) Int. Cl.: C12P 13/08, C12N 1/21

(54) **METHOD FOR PRODUCING L-THREONINE USING BACTERIA BELONGING TO THE GENUS ESCHERICHIA**
VERFAHREN ZUR HERSTELLUNG VON L-THREONIN UNTER VERWENDUNG VON ZUR GATTUNG ESCHERICHIA GEHÖRENDEN BAKTERIEN
PROCEDE POUR PRODUIRE DE LA L-THREONINE AU MOYEN DE BACTERIES APPARTENANT AU GENRE ESCHERICHIA

(30) Priority: 12.02.2004 RU 2004103986; 13.08.2004 US 601144 P
(43) Date of publication of application: 15.11.2006
(73) Proprietor: Ajinomoto Co., Inc., Tokyo 104-8315 (JP)
(72) Inventor: AKHVERDIAN, Valery, Zavenovich, Moscow 117593 (RU); SAVRASOVA, Ekaterina, Alekseevna, Moscow 107370 (RU); SAMSONOVA, Natalia, Nikolaevna, Moscow 117186 (RU); ERMISHEV, Vladimir, Yurievich, Moscow 127644 (RU); ALTMAN, Irina, Borisovna, Moscow 119435 (RU); PTITSYN, Leonid, Romanovich, Moscow 115404 (RU)
(74) Representative: HOFFMANN EITLE
(86) International application number: PCT/JP2005/002372
(87) International publication number: WO 2005/078113

(56) References cited:
- WO-A-03/008600
- WO-A-2004/090149
- DE-A1- 10 135 053
- HERMANN T ET AL: "IMPROVED L-THREONINE PRODUCTION WITH ESCHERICHIA COLI" PROCEEDINGS OF EUROPEAN CONGRESS BIOTECHNOLOGY, 24 August 2003 (2003-08-24), page 85, XP002267870
- LEE J-H ET AL: "GLOBAL ANALYSES OF TRANSCRIPTOMES AND PROTEOMES OF A PARENT STRAIN AND AN L-THREONINE-OVERPRODUCING MUTANT STRAIN" JOURNAL OF BACTERIOLOGY, WASHINGTON, DC, US, vol. 185, no. 18, September 2003 (2003-09), pages 5442-5451, XP009022136 ISSN: 0021-9193
- DANDEKAR THOMAS ET AL: "Pathway alignment: Application to the comparative analysis of glycolytic enzymes" BIOCHEMICAL JOURNAL, vol. 343, no. 1, 1 October 1999 (1999-10-01), pages 115-124, XP002330205 ISSN: 0264-6021

## Description

### Technical field

The present invention relates to biotechnology, specifically to a method for producing L-Threonine by fermentation, and more specifically to genes derived from the bacterium *Escherichia coli.* The genes are useful for improvement of productivity of L-threonine.

### Background art

Conventionally, L-amino acids have been industrially produced by methods of fermentation utilizing strains of microorganism obtained from natural sources or mutants of the same, especially modified to enhance L-amino acid productivity.

Enhancement of L-amino acid productivity has been accomplished, for example, by amplification of biosynthetic genes by transformation of a microorganism with recombinant DNA (see, for example, US patent No. 4,278,765). These techniques are based on increasing the activities of the enzymes involved in amino acid biosynthesis, and/or desensitizing the target enzymes to feedback inhibition by the produced L-amino acid or its by-products (see, for example, US patent Nos. 4,346,170, 5,661,012 and 6,040,160).

Various strains used for production of L-threonine by fermentation are known. There are strains with increased activities of the enzymes involved in L-threonine biosynthesis (US patents 5,175,107; 5,661,012; 5,705,371; 5,939,307; EP0219027), strains resistant to some chemicals such as L-threonine and its analogs (WO0114525A1, EP301572A2, US 5,376,538), strains with the target enzymes desensitized to feedback inhibition by the produced L-amino acid of its by-products (US patents 5,175,107; 5,661,012), strains with inactivated threonine degradation enzymes (US patents 5,939,307; 6,297,031).

The known threonine-producing strain VKPM B-3996 (US patents 5,175,107, and 5,705,371) is currently the best known threonine producer. For construction of the strain VKPM B-3996, several mutations and a plasmid, described below, were introduced in the parent strain *E. coli* K-12 (VKPM B-7). Mutant *thrA* gene (mutation *thrA*442) encodes aspartokinase homoserine dehydrogenase I, which imparts resistance to feedback inhibition by threonine. Mutant *ilvA* gene (mutation *ilvA*442) encodes threonine deaminase which has a low activity, leading to a low rate of isoleucine biosynthesis and a leaky phenotype of isoleucine starvation. In bacteria with *ilvA*442 mutation, transcription of *thrABC* operon is not repressed by isoleucine and therefore is very efficient for threonine production. Inactivation of *tdh* gene leads to prevention of the threonine degradation. The genetic determinant of saccharose assimilation (*scrKYABR* genes) was transferred to said strain. To increase expression of genes controlling threonine biosynthesis, plasmid pVIC40 containing mutant threonine operon *thrA442BC* was introduced in the intermediate strain TDH6. The amount of L-threonine accumulated during fermentation of the strain reaches up to 85 g/l.

The present inventors obtained, with respect to *E*. *coli* K-12, a mutant having a mutation *thrR* (herein referred to as *rhtA*23) that imparts resistance to high concentrations of threonine or homoserine in a minimal medium (Astaurova, O.B. et al., Appl. Bioch. And Microbiol., 21, 611-616 (1985)). This mutation also improved the production of L-threonine (SU Patent No. 974817), homoserine and glutamate (Astaurova, O.B. et al., Appl. Bioch. And Microbiol., 27, 556-561, 1991, EP 1013765 A) by the respective *E. coli* producing strain, such as the strain VKPM-3996. Furthermore, the present inventors have revealed that the *rhtA* gene exists at 18 min on *E. coli* chromosome close to the *glnHPQ* operon that encodes components of the glutamine transport system, and that the *rhtA* gene is identical to ORF1 (*ybiF* gene, numbers 764 to 1651 in the GenBank accession number AAA218541, gi:440181), located between *pexB* and *ompX* genes (US Patent Application Publication Nos. 2003/148473, 2003/157667). The unit expressing a protein encoded by the ORF1 has been designated as *rhtA* (rht: resistance to homoserine and threonine) gene. Also, the present inventors have found that the *rhtA*23 mutation is an A-for-G substitution at position -1 with respect to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457, EP 1013765 A).

Under conditions whereby the mainstream threonine biosynthetic pathway is studied and optimized to a great extent, the further improvement of threonine-producing strains can be done by improving the efficiency of the central metabolism pathways, such as glycolysis (Embden-Meyerhof pathway), which generates energy and various precursors of metabolites.

The glycolytic pathway includes the following enzymes: glucokinase (EC 2.7.1.2) coded by *glk* gene, phosphoglucose isomerase (EC 5.3.1.9) coded by *pgi* gene, phosphofructokinase-1 (fructose-6-P 1-kinase) (EC 2.7.1.11) coded by *pfkA* gene, fructose-1,6-bisphosphate aldolase (EC 4.1.2.13) coded by *fbaA* gene, triose-phosphate isomerase (EC 5.3.1.1) coded by *tpiA* gene, glyceraldehyde-3-phosphate dehydrogenase (EC 1.2.1.12) coded by *gapA* gene, phosphoglycerate kinase (EC 2.7.2.3) coded by *pgk* gene, phosphoglycerate mutase (EC 2.7.5.3) coded by *gpmA* gene, enolase (EC 4.2.1.11) coded by *eno* gene and isoenzymes of pyruvate kinase (EC 2.7.1.40) coded by *pykA* and *pykF* genes (Escherichia coli and Salmonella, Second Edition, Editor in Chief: F.C.Neidhardt, ASM Press, Washington D.C., 1996).

The process for production of L-lysine or other feed additives containing L-lysine by fermentation of coryneform bacteria in which alleles of the endogenous *glk* gene are overexpressed under conditions suitable for the formation of the *glk* gene product glucokinase has been disclosed (PCT application WO03054198A1). A method for the production of shikimic acid and derivatives thereof by *E. coli* having the ability to convert the carbon source to shikimic acid and transformed with recombinant DNA comprising a gene encoding a glucose facilitator protein and a glucokinase from *Zymomonas mobilis* has also been disclosed (PCT application WO0229078A2). Also, processes for the microbial preparation of intracellular metabolic intermediates, in particular erythrose 4-phosphate, and alternative processes for the microbial preparation of substances, in particular aromatic amino acids such as L-phenylalanine, in which the activity of a transaldolase is increased in a microorganism producing these substances has been disclosed (US patent 6,316,232). The '232 patent discloses as preferred embodiments that the activity of a transketolase or the activity of a transport protein for the PEP-independent uptake of a sugar and/or the activity of a glucokinase are/is additionally increased. Microorganisms employed include those belonging to the genus *Escherichia, Serratia, Bacillus, Corynebacterium* or *Breibacterium.*

A method for producing L-amino acids, particularly L-lysine, comprising culturing an altered bacterial cell, particularly *Corynebacterium glutamicum,* having an increased amount of NADPH as compared to an unaltered bacterial cell, wherein said altered bacterial cell has increased carbon flux through the oxidative branch of the pentose phosphate pathway have been disclosed (PCT application WO0107626A2). This publication discloses as the preferred embodiment of the method an altered bacterial cell which has decreased carbon flux via the glycolytic pathway due to a decreased amount of 6-phosphoglucose isomerase enzymatic activity, which results from a mutation in the *pgi* gene. A similar method for producing L-lysine using coryneform bacteria having intracellular activity of a phosphoglucose isomerase (pgi) enzyme eliminated has also been disclosed (US patent 6,586,214).

A method for producing L-lysine comprising cultivating L-lysine-producing coryneform bacterium in which the intracellular activity of 6-phosphofructokinase coded by *pfkA* gene is increased has been disclosed (European patent application EP1195431A1). At the same time, a process for the fermentative preparation of L-amino acids, in particular L-lysine, comprising culturing coryneform bacteria to produce the desired L-amino acid, in which at least the gene coding for 6-phosphofructokinase (*pfkA* gene) and/or the gene coding for 1-phosphofructokinase (*fruK* gene) are/is attenuated has been disclosed (PCT application WO02074944A1). This publication discloses as the preferred embodiment of the process the preparation of L-lysine using coryneform bacterium in which, in addition to attenuation of *pfkA* and/or *fruK* genes, one or more of the genes selected from the group comprising the *lysC* gene coding for a feedback-resistant aspartate kinase, the *dapA* gene coding for dihydrodipicolinate synthase, the *gap* gene coding for glyceraldehyde phosphate dehydrogenase, the *pyc* gene coding for pyruvate carboxylase, the *mqo* gene coding for malate:quinone oxidoreductase, the *zwf* gene coding for glucose phosphate dehydrogenase, the *lysE* gene coding for lysine exporter, the *zwa*1 gene coding for the zwa1 protein, the gene *tpi* coding for triose phosphate isomerase, and the *pgk* gene coding for 3-phosphoglycerate kinase is/are simultaneously enhanced, and, in particular, overexpressed.

A process for the preparation of L-amino acids, such as L-lysine and L-threonine, by fermentation of coryneform bacteria, in which at least the *zwf* gene is amplified, has been described (PCT application WO0170995A1). This publication discloses as the preferred embodiment of the process the preparation of the L-amino acids using coryneform bacterium in which, in addition to attenuation of *zwf* gene, one or more of the genes selected from the group comprising *dapA* gene which codes for dihydrodipicolinate synthase, the *lysC* gene which codes for a feed back resistant aspartate kinase, the *gap* gene which codes for glycerolaldehyde-3-phosphate dehydrogenase, the *pyc* gene which codes for pyruvate carboxylase, the *tkt* gene which codes for transketolase, the *gnd* gene which codes for gluconate 6-phosphate dehydrogenase, the *lysE* gene which codes for lysine exporter, the *zwa*1 gene, the *eno* gene which codes for enolase is/are amplified or over-expressed at the same time.

Coryneform bacteria which produce L-amino acids, including L-threonine, having at least one copy presented at the natural site (locus), and up to three additional copies of open reading frames (ORF) chosen from a group including, among others, *eno, gap, pgk* and *tpi* genes, have been disclosed (PCT applications WO03014330A2, WO03040373A2).

A process for preparing L-glutamic acid by fermentation of coryneform bacteria in which the nucleotide sequence coding for D-alanine racemase (alr) is attenuated, and in particular, eliminated, has been disclosed (PCT application WO0208437A2). This publication discloses as the preferred embodiment of the process the preparation of L-glutamic acid using coryneform bacterium in which, in addition to attenuation of the nucleotide sequence coding for D-alanine racemase (alr), one or more of the genes selected from the group including, among others, the *gap* and *eno* genes are enhanced, and in particular, over-expressed.

A method for producing fine chemicals or metabolites, such as L-threonine, using microorganisms, in particular, coryneform bacteria or *Escherichia coli,* in which the phosphorylatability of at least one protein has been permanently altered such that the biosynthesis of at least one fine chemical synthesized by the microorganism is increased compared to the wild-type due to a mutation in at least one amino acid of the protein, has been disclosed (PCT application WO03023016A2). One example of such protein is mutant enolase (enoS330E).

Among genes coding for enzymes of glycolytic pathway, four genes have been used for improvement of L-threonine production using bacterium of *Enterobacteriaceae* family, particularly *Escherichia coli.* There are *fbaA, gpmA* (*pgm*)*, pykF* and *pfkB* genes.

Thus, process for the preparation of L-amino acids, in particular L-threonine, by fermentation of *Enterobacteriaceae* family microorganisms which produce the desired L-amino acid and in which the *fba* gene or the nucleotide sequence which codes for this gene is enhanced, in particular, over-expressed, has been disclosed (PCT application WO03004664A2). At the same time, a process for the fermentative preparation of L-amino acids, in particular L-threonine, by fermentation of microorganisms of the *Enterobacteriaceae* family which produce the desired L-amino acid and in which one or more of the genes chosen from the group comprising, among others, *fba* gene or nucleotide sequences which code for these, is/are attenuated, in particular eliminated, has been disclosed by the same applicant in the PCT application WO03004662A2, but it contains no examples.

Also, a process for the preparation of L-amino acids, in particular L-threonine, by fermentation of microorganisms of the *Enterobacteriaceae* family which produce the desired Lamino acid and in which the *pgm* gene or the nucleotide sequence for this is enhanced, in particular, over-expressed, has been disclosed (PCT application WO03004598A2). At the same time, the process for the fermentative preparation of L-amino acids, in particular L-threonine, by fermentation of microorganisms of the *Enterobacteriaceae* family which produce the desired L-amino acid and in which one or more of the genes chosen from the group comprising among others *pgm* gene or nucleotide sequences for these is/are attenuated, in particular, eliminated, has been disclosed by the same applicant in the PCT application WO03004662 but it contains no examples.

And, the process for the preparation of L-amino acids, in particular L-threonine, by fermentation of microorganisms of the *Enterobacteriaceae* family which produce the desired L-amino acid and in which the *pykF* gene or the nucleotide sequence for this is enhanced, in particular, over-expressed, has been disclosed (PCT application WO03008609A2). At the same time, a process for the fermentative preparation of L-amino acids, in particular L-threonine, by fermentation of microorganisms of the *Enterobacteriaceae* family which produce the desired L-amino acid and in which one or more of the genes chosen from the group comprising, among others, *pykF* gene or nucleotide sequences which code for these is/are attenuated, in particular, eliminated, has been disclosed by the same applicant in the PCT application WO03008600A2, but it contains no examples.

And finally, a process for the preparation of L-amino acids, in particular L-threonine, by fermentation of microorganisms of the *Enterobacteriaceae* family which produce the desired L-amino acid and in which the *pfkB* gene or the nucleotide sequence which codes for this is enhanced, in particular over-expressed, was disclosed (PCT application WO03008610A2). At the same time, process for the fermentative preparation of L-amino acids, in particular L-threonine, by fermentation of microorganisms of the *Enterobacteriaceae* family which produce the desired L-amino acid and in which one or more of the genes chosen from the group comprising among others *pfkB* gene or nucleotide sequences which code for these, is (are) attenuated, in particular eliminated, was claimed by the same applicant in the PCT application WO03008600A2 containing no examples.

There have been no disclosures to date of using bacterium belonging to the genus *Escherichia* with enhanced expression of genes coding for enzymes of glycolytic pathway, such as *glk, pgi, pfkA, tpiA, gapA, pgk, eno* and *pykA,* for production of L-threonine.

### Disclosure of the invention

An object of present invention is to enhance the productivity of L-threonine-producing Escherichia coli strains and to provide a method for producing L-threonine using these strains.

This aim was achieved by the finding that genes, such as *glk, pgi, pfkA, tpiA, gapA, pgk, eno* and *pykA,* which code for enzymes of the glycolytic pathway, upon being cloned on a low copy vector, enhance L-threonine production of L-threonine-producing strains when the strain is transformed with the plasmid harboring the gene. Thus the present invention has been completed.

An L-threonine-produding bacterium belonging to the genus *Escherichia* is described wherein the bacterium has been modified to enhance an activity of one or more of glycolytic enzymes.

An L-threonine producing bacterium belonging to the genus *Escherichia* is described wherein the bacterium has been modified to enhance expression of one or more of the genes chosen from the group consisting of *glk, pgi, pfkA, tpiA, gapA, pgk, eno* and *pykA,* which code for enzymes of glycolytic pathway, or the nucleotide sequences, which code for these.

The bacterium as described above, is further described wherein the expression of one or more of the genes is enhanced by increasing copy number of the gene(s), or modifying an expression control sequence of the gene(s) so that the expression of the gene(s) is enhanced.

The bacterium as described above, is further described wherein the copy number is increased by transformation of the bacterium with a low copy vector containing the gene or genes.

The bacterium as described above, is further described wherein the genes are originated from a bacterium belonging to the genus *Escherichia.*

The bacterium as described above, is further described wherein the bacterium has been further modified to enhance expression of one or more genes selected from the group consisting of:
- the mutant *thrA* gene which codes for aspartokinase homoserine dehydrogenase I resistant to feed back inhibition by threonine;
- the *thrB* gene which codes for homoserine kinase;
- the *thrC* gene which codes for threonine synthase;
- the *rhtA* gene, which codes for putative transmembrane protein.

The bacterium as described above, is further described wherein the bacterium has been modified to increase expression amount of the mutant *thrA* wherein the bacterium has been modified to increase expression amount of the mutant *thrA* gene, the *thrB* gene, the *thrC* gene and the *rhtA* gene.

And it is a further object of the invention to provide a method for producing threonine, which comprises cultivating the Escherichia coli bacterium as described above in a culture medium to produce and accumulate L-threonine in the culture medium, and collecting the L-threonine from the culture medium.

### Brief Description of Drawings

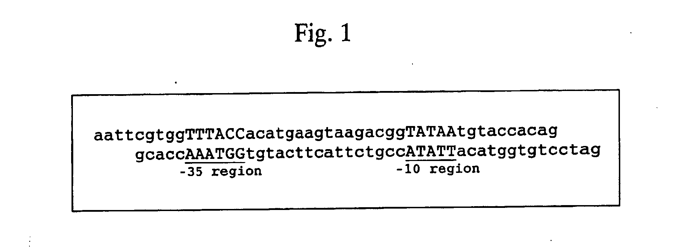

Figure 1 shows the structure of synthetic mutant promoter P_{A3m}.

### Description of the Preferred Embodiments

The bacterium used in the present invention is an Escherichia coli L-Threonine-producing bacterium wherein the bacterium has been modified to enhance expression of one or more of the genes chosen from the group consisting of *glk, pgi, pfkA, tpiA, gapA, pgk, eno* and *pykA,* which code for enzymes of glycolytic pathway, or the nucleotide sequences, which code for these.

In the present invention, "L-threonine-producing bacterium" means a bacterium, which has an ability to cause accumulation of L-threonine in a medium, when the bacterium of the present invention is cultured in the medium. The L-threonine-producing ability may be imparted or enhanced by breeding. The phrase "L-threonine-producing bacterium" as used herein also means a bacterium, which is able to produce and cause accumulation of L-threonine in a culture medium in amount larger than a wild type or parental strain of *E*. *coli,* such as *E*. *coli* K-12 strain.

The phrase "a bacterium belonging to the genus *Escherichia"* means that the bacterium is classified as the genus *Escherichia* according to the classification known to a person skilled in the art of microbiology. Examples of a microorganism belonging to the genus *Escherichia* as used in the present invention include, but are not limited to, *Escherichia coli* (*E. coli*).

The bacterium that can be used in the present intention is Escherichia coli described by Neidhardt, F.C. et al. (Escherichia coli and Salmonella typhimurium, American Society for Microbiology, Washington D.C., 1208, Table 1).

The phrase "modified to enhance expression of-gene(s)" means that the expression amount of the gene(s) is higher than that of a non-modified strain, for example, a wild-type strain. Examples of such modifications include increasing the number of gene(s) to be expressed per cell, increasing the expression level of the gene, and so forth. The quantity of the copy number of the expressed gene is measured, for example, by restriction of the chromosomal DNA, followed by Southern blotting using a probe constructed based on the gene sequence, fluorescence *in situ* hybridization (FISH), and the like. The level of gene expression is measured by different methods, including Northern blotting, quantitative RT-PCR and the like. Furthermore, the *Escherichia coli* K-1, for example, can be used as a wild-type strain for comparison. As a result of the enhancement of expression of the genes(s), the amount of L-threonine which accumulates in a medium increases.

Enzymes of glycolytic pathway according to the present invention are presented by glucokinase (EC 2.7.1.2) coded by *glk* gene, phosplioglucose isomerase (EC 5.3.1.9) coded by *pgi* gene, phosphofructokinase-1 (fructose-6-P 1-kinase) (EC 2.7.1.11) coded by *pfkA* gene, triose-phosphate isomerase (EC 5.3.1.1) coded by *tpiA* gene, glyceraldehyde-3-phosphate dehydrogenase (GAPDH) (EC 1.2.1.12) coded by *gapA* gene, phosphoglycerate kinase (EC 2.7.2.3) coded by *pgk* gene, enolase (EC 4.2.1.11) coded by *eno* gene and isoenzymes of pyruvate kinase (EC 2.7.1.40) coded by *pykA* gene.

In the present invention, the term "glucokinase" means an enzyme capable of catalyzing the reaction of ATP-dependent phosphorylation of glucose with formation of glucose-6-phosphate. The term "phosphoglucose isomerase" means an enzyme capable of catalyzing the reaction of conversion of glucose-6-phosphate into fructose-6phosphate. The term "phosphofructokinase-1 (fructose-6-P 1-kinase)" means an enzyme capable of catalyzing the reaction of ATP-dependent phosphorylation of glucose-6-phosphate with formation of glucose-1,6-diphosphate. The term "triose-phosphate isomerase " means an enzyme which interconverts dihydroxyacetone phosphate and glyceraldehyde-3-phosphate. The term "glyceraldehyde-3-phosphate dehydrogenase" means an enzyme capable of catalyzing the reaction of NAD⁺-dependent conversion of glyceraldehyde-3-phosphate into 1,3-diphosphoglycerate. The term "phosphoglycerate kinase" means an enzyme capable of catalyzing the reaction of dephosphorylation of 1,3-diphosphoglycerate with release of 3-phosphoglucerate and ATP. The term "enolase" means an enzyme capable of catalyzing the reaction of dehydration of 2-phosphoglucerate with release of phosphoenolpyruvate. The term "pyruvate kinase" means an enzyme capable of catalyzing the reaction of formation of pyruvate from phosphoenolpyruvate with release of ATP.

Any genes derived from bacteria belonging to the genus *Escherichia* and genes derived from other bacteria such as coryneform bacteria, bacteria belonging to the genus *Bacillus* or the like can be used as the genes coding for the glycolytic enzymes. Among these, genes derived from bacteria belonging to the genus *Escherichia* are preferred.

As the gene coding for glucokinase of *Escherichia coli, glk* gene has been elucidated (nucleotide numbers 2506481 to 2507446 in the sequence of GenBank accession NC_000913.1, gi:16130320; SEQ ID NO: 1). The *glk* gene is located between b2387 and b2389 ORFs on the chromosome of E. *coli* strain K12. As the gene coding for phosphoglucose isomerase of *Escherichia coli, pgi* gene has been elucidated (nucleotide numbers 4231337 to 4232986 in the sequence of GenBank accession NC_000913.1, gi:16131851; SEQ ID NO: 3). The *pgi* gene is located between *lysC* gene and *jbE* on the chromosome of *E*. *coli* strain K12. As the gene coding for phosphofructokinase-1 of *Escherichia coli, pjkA* gene has been elucidated (nucleotide numbers 4105132 to 4106094 in the sequence of GenBank accession NC_000913.1, gi:16131754; SEQ ID NO: 5). The *pfkA* gene is located between *yiiP* ORF and *sbp* gene on the chromosome of *E. coli* strain K12. As the gene coding for triose-phosphate isomerase of *Escherichia coli, tpiA* gene has been elucidated (nucleotide numbers 4108320 to 4109087 in the sequence of GenBank accession NC_000913.1, gi:16131757; SEQ ID NO: 9). The *tapiA* gene is located between *cdh* gene and *yiiQ* ORF on the chromosome of *E*. *coli* strain K12. As the gene coding for glyceraldehyde-3-phosphate dehydrogenase of *Escherichia coli, gapA* gene has been elucidated (nucleotide numbers 1860795 to 1861790 in the sequence of GenBank accession NC_000913.1, gi:16129733; SEQ ID NO: 11). The *gapA* gene is located between *yeaA* and *yeaD* ORFs on the chromosome of E. *coli* strain K12. As the gene coding for phosphoglycerate kinase of *Escherichia coli, pgk* gene has been elucidated (nucleotide numbers 3069479 to 3070642 in the sequence of GenBank accession NC_000913.1, gi:16130827; SEQ ID NO: 13). The *pgk* gene is located between *fbaA* and *epd* genes on the chromosome of *E*. *coli* strain K12. As the gene coding for enolase of *Escherichia coli, eno* gene has been elucidated (nucleotide numbers 2904665 to 2905963 in the sequence of GenBank accession NC_000913.1, gi:16130686; SEQ ID NO: 17). The *eno* gene is located between b2778 ORF and *pyrG* gene on the chromosome of *E*. *coli* strain K12. As the gene coding for pyruvate kinase II of *Escherichia coli, pykA* gene has been elucidated (nucleotide numbers 1935673 to 1937115 and 1753722 to 1755134 in the sequence of GenBank accession NC_000913.1, gi:16129807 and gi:16129632; SEQ ID NOs: 19 and 21, respectively). The *pykA* gene is located between *yebK* ORF and *msbB* gene on the chromosome of *E*. *coli* strain K12. Therefore, the aforementioned genes can be obtained by PCR (polymerase chain reaction; refer to White, T.J. et al., Trends Genet., 5, 185 (1989)) utilizing primers prepared based on the reported nucleotide sequence of the genes.

The *glk* gene originated from *Escherichia coli* is exemplified by a DNA which encodes the following protein (A) or (B):
(A) a protein, which comprises the amino acid sequence shown in SEQ ID NO: 2; or
(B) a protein which comprises an amino acid sequence including deletion, substitution, insertion or addition of one or several amino acids in the amino acid sequence shown in SEQ ID NO: 2, and which has an activity of glucokinase.
   The *pgi* gene originated from *Escherichia coli* is exemplified by a DNA which encodes the following protein (C) or (D):
(C) a protein, which comprises the amino acid sequence shown in SEQ ID NO: 4; or
(D) a protein which comprises an amino acid sequence including deletion, substitution, insertion or addition of one or several amino acids in the amino acid sequence shown in SEQ ID NO: 4, and which has an activity of phosphoglucose isomerase.
   The *pfkA* gene originated from *Escherichia coli* is exemplified by a DNA which encodes the following protein (E) or (F):
(E) a protein, which comprises the amino acid sequence shown in SEQ ID NO: 6; or
(F) a protein which comprises an amino acid sequence including deletion, substitution, insertion or addition of one or several amino acids in the amino acid sequence shown in SEQ ID NO: 6, and which has an activity of phosphofructokinase-1 (fructose-6-P 1-kinase).
   The *tpiA* gene originated from *Escherichia coli* is exemplified by a DNA which encodes the following protein (G) or (H):
(G) a protein, which comprises the amino acid sequence shown in SEQ ID NO: 8; or
(H) a protein which comprises an amino acid sequence including deletion, substitution, insertion or addition of one or several amino acids in the amino acid sequence shown in SEQ ID NO: 8, and which has an activity of triose-phosphate isomerase.
   The *gapA* gene originated from *Escherichia coli* is exemplified by a DNA which encodes the following protein (I) or (J):
(I) a protein, which comprises the amino acid sequence shown in SEQ ID NO: 10; or
(J) a protein which comprises an amino acid sequence including deletion, substitution, insertion or addition of one or several amino acids in the amino acid sequence shown in SEQ ID NO: 10, and which has an activity of glyceraldehyde-3-phosphate dehydrogenase.
   The *pgk* gene originated from *Escherichia coli* is exemplified by a DNA which encodes the following protein (K) or (L):
(K) a protein, which comprises the amino acid sequence shown in SEQ ID NO: 12; or
(L) a protein which comprises an amino acid sequence including deletion, substitution, insertion or addition of one or several amino acids in the amino acid sequence shown in SEQ ID NO: 12, and which has an activity of phosphoglycerate kinase.
   The *eno* gene originated from *Escherichia coli* is exemplified by a DNA which encodes the following protein (M) or (N):
(M) a protein, which comprises the amino acid sequence shown in SEQ ID NO: 14; or
(N) a protein which comprises an amino acid sequence including deletion, substitution, insertion or addition of one or several amino acids in the amino acid sequence shown in SEQ ID NO: 14, and which has an activity of enolase.
   The *pykA* gene originated from *Escherichia coli* is exemplified by a DNA which encodes the following protein (O) or (P):
(O) a protein, which comprises the amino acid sequence shown in SEQ ID NO: 16; or
(P) a protein which comprises an amino acid sequence including deletion, substitution, insertion or addition of one or several amino acids in the amino acid sequence shown in SEQ ID NO: 16, and which has an activity of pyruvate kinase.

The number of "several" amino acids differs depending on the position or the type of amino acid residues in the three dimensional structure of the protein. It may be, for example, 2 to 30, preferably 2 to 15, and more preferably 2 to 5 for the protein (A). This is because some amino acids have high homology to one another so the three dimensional structure of the protein or its activity is not affected by such change. Therefore, the protein (B) may be one which has homology of not less than 30 to 50 %, preferably 50 to 70 %, and more preferably between 70 to 90%, still more preferably greater than 90%, and most preferably greater than 95%, with respect to the entire amino acid sequence constituting glucokinase, and which has the activity of glucokinase. The same approach is applied to other proteins (C), (E), (G), (I), (K), (M) and (O).

The DNAs, which encodes for the substantially the same proteins as each of enzyme of glycolytic pathway described above, are obtained, for example, by modifying the nucleotide sequence of DNA encoding for the enzyme, for example, by means of the site-directed mutagenesis method so that one or more amino acid residues at a specified site involve deletion, substitution, insertion, or addition. DNA modified as described above is obtained by conventionally known mutation treatments. Such treatments include hydroxylamine treatment of the DNA encoding for proteins of present invention or treatment of the bacterium containing the DNA with UV irradiation or a reagent such as N-methyl-N'-nitro-N-nitrosoguanidine or nitrous acid.

A DNA encoding for substantially the same protein as glucokinase is obtained by expressing a DNA having the mutation as described above in an appropriate cell, and investigating the activity of any expressed product. A DNA coding for substantially the same protein as glucokinase can also be obtained by isolating a DNA that is hybridizable with a probe having a nucleotide sequence which contains, for example, the nucleotide sequence shown in SEQ ID NO: 1, under the stringent conditions, and codes for a protein having the activity of glucokinase, from DNA coding for glucokinase having a mutation or from a cell harboring it. The "stringent conditions" referred to herein are conditions under which so-called specific hybrids are formed, and non-specific hybrids are not formed. It is difficult to clearly express this condition by using any numerical value. However, for example, the stringent conditions can be exemplified by conditions under which DNAs having high homology, for example, DNAs having homology of not less than 50%, preferably 50 to 70 %, and more preferably between 70 to 90%, still more preferably greater than 90%, and most preferably greater than 95%, are able to hybridize with each other, but DNAs having homology lower than the above are not able to hybridize with each other.

To evaluate degree of protein or DNA homology several calculation methods, such as BLAST search, FASTA search and CrustalW, can be used. BLAST (Basic Local Alignment Search Tool) is the heuristic search algorithm employed by the programs blastp, blastn, blastx, megablast, tblastn, and tblastx; these programs ascribe significance to their findings using the statistical methods of Karlin, Samuel and Stephen F. Altschul ("Methods for assessing the statistical significance of molecular sequence features by using general scoring schemes". Proc. Natl. Acad. Sci. USA, 1990, 87:2264-68; "Applications and statistics for multiple high-scoring segments in molecular sequences". Proc. Natl. Acad. Sci. USA, 1993, 90:5873-7). The FASTA search method is described by W. R. Pearson ("Rapid and Sensitive Sequence Comparison with FASTP and FASTA", Methods in Enzymology, 1990 183:63- 98). ClustalW method is described by Thompson J.D., Higgins D.G. and Gibson T.J. ("CLUSTAL W: improving the sensitivity of progressive multiple sequence alignment through sequence weighting, position-specific gap penalties and weight matrix choice", Nucleic Acids Res. 1994, 22:4673-4680).

Alternatively, the stringent conditions may be exemplified by conditions under which DNAs are hybridized with each other at a salt concentration equivalent to ordinary washing conditions in Southern hybridization, i.e., 1 x SSC, 0.1% SDS, preferably 0.1 x SSC, 0.1% SDS, at 60°C. Duration of washing procedure depends on the type of membrane used for blotting and, as a rule, is recommended by manufacturer. For example, recommended duration of washing the Hybond^{™} N+ nylon membrane (Amersham) under stringent conditions is 15 minutes. Preferably, washing may be performed 2 to 3 times.

A partial sequence of the nucleotide sequence of SEQ ID NO: 1 can also be used as a probe. Probes may be prepared by PCR using primers produced based on the nucleotide sequence of SEQ ID NO: 1 as primers, and a DNA fragment containing the nucleotide sequence of SEQ ID NO: 1 as a template. When a DNA fragment having a length of about 300 bp is used as the probe, the washing conditions may include, for example, 50°C, 2 x SSC and 0.1% SDS.

The substitution, deletion, insertion, or addition of nucleotides as described above also includes mutation, which naturally occurs (mutant or variant), for example, due to variety of species or genus of bacterium, which contains glucokinase.

A DNA coding for substantially the same proteins as the other enzymes of glycolytic pathway are obtained similarly to glucokinase as described above.

"Transformation of a bacterium with DNA encoding a protein" means introduction of the DNA into a bacterium cell, for example, by conventional methods. Transformation of a bacterial cell with this DNA will result in an increase in expression of the gene encoding the protein of present invention and will enhance the activity of the protein in the bacterial cell. Methods of transformation include any known methods that have hitherto been reported. For example, a method of treating recipient cells with calcium chloride so as to increase permeability of the cells to DNA has been reported for *Escherichia coli* K-12 (Mandel, M. and Higa, A., J. Mol. Biol., 53, 159 (1970)) and may be used.

Methods of gene expression enhancement include increasing the gene copy number. Introduction of a gene into a vector that is able to function in a bacterium belonging to the genus *Escherichia* increases the copy number of the gene. Preferably, low copy vectors are used. The low-copy vector is exemplified by pSC101, pMW118, pMW119 and the like. The term "low copy vector" is used for vectors, the copy number of which is up to 5 copies per cell.

Enhancement of gene expression may also be achieved by introduction of multiple copies of the gene into a bacterial chromosome by, for example, a method of homologous recombination, Mu integration or the like. For example, one round of Mu integration allows to introduce into bacterial chromosome up to 3 copies of the gene.

Enhancement of gene expression may also be achieved by modifying an expression control sequence of the gene so that the expression of the gene is enhanced, for example, by placing the DNA of the present invention under the control of a potent promoter. For example, the *lac* promoter, the *trp* promoter, the *trc* promoter, the P_{R} or the P_{L} promoters of lambda phage are known as potent promoters. Strength of promoter is defined by frequency of acts of the RNA synthesis initiation. Method for evaluation the strength of promoter described by, for example, Deuschle U., Kammerer W., Gentz R., Bujard H. (Promoters in Escherichia coli: a hierarchy of in vivo strength indicates alternate structures. EMBO J., 5, 2987-2994 (1986)).

Use of a potent promoter can be combined with multiplication of gene copies.

Alternatively, a promoter can be enhanced by, for example, introducing a mutation into the promoter to increase the transcription level of a gene located downstream of the promoter. Further, it is known that substitution of several nucleotides in a spacer between the ribosome binding site (RBS) and the start codon and especially the sequences immediately upstream of the start codon profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629, 1984). Earlier, the authors of present invention showed, the *rhtA23* mutation is an A-for-G substitution at position -1 with respect to the ATG start codon (ABSTRACTS of 17^{th} International Congress of Biochemistry and Molecular Biology in conjugation with 1997 Annual Meeting of the American Society for Biochemistry and Molecular Biology, San Francisco, California August 24-29, 1997, abstract No. 457). Therefore, it may be suggested that *rhtA23* mutation enhances the *rhtA* gene expression and, as a consequence, increases the level of resistance to threonine, homoserine and some other substances transported out of cells.

Moreover, it is also possible to introduce nucleotide substitution into a promoter region of the one or more of genes of glycolysis on the bacterial chromosome so that it should be modified into a stronger one. The alteration of expression control sequence can be performed, for example, in the same manner as the gene substitution using a temperature sensitive plasmid, as disclosed in International Patent Publication WO00/18935 and Japanese Patent Publication No. 1-215280.

Increasing the copy number of one or more of genes coding for enzymes of glycolytic pathway can also be achieved by introducing multiple copies of the gene into chromosomal DNA of bacterium. To introduce multiple copies of a gene into a bacterial chromosome, homologous recombination is carried out by using a sequence whose multiple copies exist in the chromosomal DNA as targets. As sequences whose multiple copies exist in the chromosomal DNA, repetitive DNA, inverted repeats existing at the end of a transposable element can be used. Also, as disclosed in Japanese Patent Laid-open No. 2-109985, it is possible to incorporate the concrete gene into transposon, and allow it to be transferred to introduce multiple copies of the gene into the chromosomal DNA.

Methods for preparation of plasmid DNA, digestion and ligation of DNA, transformation, selection of an oligonucleotide as a primer and the like may be ordinary methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

The bacterium of the present invention can be obtained by introduction of the aforementioned DNAs into bacterium which inherently has the ability to produce L-threonine. Alternatively, the bacterium of the present invention can be obtained by imparting an ability to produce L- threonine to the bacterium already containing the DNAs.

Examples of parent strains encompassed by the present invention include, but are not limited to, the threonine-producing bacteria belonging to the genus *Escherichia* such as *E. coli* strain TDH-6/pVIC40 (VKPM B-3996) (US Patent 5, 175, 107, US patent 5,705,371), *E. coli* strain NRRL-21593 (US Patent 5,939,307), *E. coli* strain FERM BP-3756 (US patent 5,474,918), *E. coli* strains FERM BP-3519 and FERM BP-3520 (US patent 5,376,538), *E. coli* strain MG442 (Gusyatiner et al., Genetika (in Russian), 14, 947-956 (1978)), *E. coli* strains VL643 and VL2055 (EP 1149911 A) and the like may be used.

The strain TDH-6 is deficient in the *thrC* gene as well as being sucrose-assimilative, and the *ilvA* gene has a leaky mutation. This strain has a mutation in the *rhtA* gene, which imparts resistance to high concentrations of threonine or homoserine. The strain B-3996 (TDH-6/pVIC40) contains the plasmid pVIC40 which had been obtained by inserting *thrA***BC* operon including mutant *thrA* gene encoding aspartokinase homoserine dehydrogenase I which has substantially desensitized feedback inhibition by threonine into RSF1010-derived vector. The strain B-3996 was deposited on November 19, 1987 in All-Union Scientific Center of Antibiotics (Nagatinskaya Street 3-A, 113105 Moscow, Russian Federation) on April 7, 1987 under the accession number RIA 1867. The strain was also deposited in Russian National Collection of Industrial Microorganisms (VKPM) (Dorozhny proezd. 1, Moscow 113545, Russian Federation) on April 7, 1987 under the accession number B-3996.

Preferably, the bacterium of the present invention is preferably further modified to enhance expression of one or more of the following genes along with one or more of genes coding for enzymes of glycolytic pathway:
- the mutant *thrA* gene which codes for aspartokinase homoserine dehydrogenase I resistant to feed back inhibition by threonine;
- the *thrB* gene which codes for homoserine kinase;
- the *thrC* gene which codes for threonine synthase;

Another preferred embodiment of the present invention is the bacterium modified to enhance the *rhtA* gene which codes for a putative transmembrane protein in addition to enhancement of gene(s) coding for glycolytic enzyme(s). The most preferred embodiment of the present invention is a bacterium modified to increase expression of the gene(s) coding for glycolytic enzyme(s), the mutant *thrA* gene, the *thrB* gene, the *thrC* gene and the *rhtA* gene.

The method for producing L-threonine of the present invention includes the steps of cultivating the bacterium of the present invention in a culture medium, allowing L-threonine to accumulate in the culture medium, and collecting L-threonine from the culture medium.

In the present invention, the cultivation, the collection and purification of L-threonine from the medium and the like may be performed in a manner similar to the conventional fermentation method wherein L-threonine is produced using a microorganism.

A medium used for culture may be either a synthetic or natural medium, so long as the medium includes a carbon source and a nitrogen source and minerals and, if necessary, appropriate amounts of nutrients which the microorganism requires for growth. The carbon source may include various carbohydrates such as glucose and sucrose, and various organic acids. Depending on the mode of assimilation of the chosen microorganism, alcohol, including ethanol and glycerol, may be used. As the nitrogen source, various ammonium salts such as ammonia and ammonium sulfate, other nitrogen compounds such as amines, a natural nitrogen source such as peptone, soybean-hydrolysate, and digested fermentative microorganism are used. As minerals, potassium monophosphate, magnesium sulfate, sodium chloride, ferrous sulfate, manganese sulfate, calcium chloride, and the like are used. As vitamins, thiamine, yeast extract and the like are used. Additional nutrients can be added to the medium if necessary. For example, if the microorganism requires isoleucine for growth (isoleucine auxotrophy), a sufficient amount of isoleucine can be added to the cultivation medium.

The cultivation is performed preferably under aerobic conditions such as a shaking culture, and stirring culture with aeration, at a temperature of 20 to 40 °C, preferably 30 to 38 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to the accumulation of the L-threonine in the liquid medium.

After cultivation, solids such as cells can be removed from the liquid medium by centrifugation or membrane filtration, and then L- threonine can be collected and purified by ion-exchange, concentration and crystallization methods.

### Examples

The present invention will be more concretely explained below with reference to the following non-limited examples. The *E. coli* strain VKPM B-3996 (US patent 5,175,107) was used as a parental strain to evaluate the effect of the amplification of genes coding for the enzymes of glycolytic pathway on L-threonine production.

The plasmid pMW119 and it derivatives are compatible with plasmid pVIC40 (replicon pRSF1010), therefore the two plasmids pVIC40 and derivative of pMW119 containing the gene coding for enzyme of glycolytic pathway could be maintained in the bacterium simultaneously. In the tables with results of fermentations, data from at least (please confirm)three independent experiments are presented.

### Example 1: Cloning of glk gene from E. coli and effect of enhanced expression of glk gene on L-threonine production.

The *glk* gene was obtained by PCR using chromosomal DNA of the *E. coli* strain MG 1655 (VKPM B-6195) as the template and primers P1 (SEQ ID NO: 17) and P2 (SEQ ID NO: 18). The strain MG1655 is available from American Type Culture Collection (ATCC700926). Primer P1 contains recognition site of *Bam*HI restrictases introduced in the 5'-end thereof. Primer P2 contains recognition site of *Sac*I restrictases introduced in the 5'-end thereof. The obtained DNA fragment (968 bp) containing the *glk* gene was treated with *Bam*HI and *Sac*I restrictases and cloned into the plasmid pMW119 previously modified to substitute promoter P*_{lac}* by promoter P_{R} of the phage lambda and then treated with the same restrictases. Thus the plasmid pMW-P_{R}-glk containing the *glk* gene under the control of promoter P_{R} was constructed. Non-regulated high level of *glk* gene expression could be achieved using this plasmid.

The pMW-P_{R}-glk plasmid was introduced into the streptomycin-resistant threonine producer *E. coli* strain B-3996 (US patent 5,175,107). Thus, the strain B-3996(pMW-P_{R}-glk) was obtained.

Both *E*. *coli* strains B-3996 and B-3996(pMW-P_{R}-glk) were grown for 18-24 hours at 37 °C on L-agar plates containing streptomycin (100 µg/ml) and ampicillin (100 µg/ml). To obtain seed culture, the strain was grown on a rotary shaker (250 rpm) at 32°C for 18 hours in 20x200 mm test tubes containing 2 ml of L-broth with 4% glucose. Then, the fermentation medium was inoculated with 0.1 ml (5%) of seed material. The fermentation was performed in 2 ml of minimal medium for fermentation in 20x200 mm test tubes. Cells were grown for 24 hours at 32 °C with shaking at 250 rpm.

After cultivation, an accumulated amount of L-threonine in the medium was determined by TLC. Sorbfil plates (Stock Company Sorbopolymer, Krasnodar, Russia) were developed with a mobile phase: propan-2-ol : acetone : water : 25% aqueous ammonia = 25 : 25: 7 : 6 (v/v). A solution (2%) of ninhydrin in acetone was used as a visualizing reagent. The results are presented in Table 1.

The composition of the fermentation medium (g/l) is as follows:

| | |
|---|---|
| Glucose | 40.0 |
| (NH₄)₂SO₄ | 10.0 |
| KH₂PO₄ | 1.0 |
| MgSO₄.7H₂O | 0.4 |
| FeSO₄.7H₂O | 0.02 |
| MnSO₄.5H₂O | 0.02 |
| Thiamine HCl | 0.0002 |
| Yeast extract | 1.0 |
| CaCO₃ | 20.0 |
| L-isoleucine | 0.05 |

Glucose and magnesium sulfate are sterilized separately. CaCO₃ dry-heat is sterilized at 180°C for 2 h. The pH is adjusted to 7.0. Antibiotics are introduced into the medium after sterilization.

**Table 1.**

| Strain | OD₅₆₀ | Threonine, g/l |
|---|---|---|
| B-3996 | 9.7±0.1 | 14.3±0.1 |
| B-3996(pM W-P_{R}-glk) | 9.6±0.1 | 14.8±0.1 |

As it is seen from the Table 1, the enhancement of *glk* gene expression improved L-threonine productivity of the strain B-3996.

Example 2: Cloning of *pfkA* gene from *E. coli* and effect of enhanced expression of *pfkA* gene on Threonine production.

The *pfkA* gene was obtained by PCR using chromosomal DNA of the *E. coli* strain MG 1655 (VKPM B-6195) as the template and primers P3 (SEQ ID NO: 19) and P4 (SEQ ID NO: 20). Primer P3 contains recognition site of *Bam*HI restrictases introduced in the 5'-end thereof. Primer P4 contains recognition site of *Sac*I restrictases introduced in the 5'-end thereof. Obtained DNA fragment (987 bp) containing *pfkA* gene was directly cloning into vector pCR 2.1 (Invitrogen) by overnight ligation at + 4 °C. Then *Bam*HI - *Sac*I DNA fragment containing *pfkA* gene was recloned into the plasmid pMW119 previously modified to substitute promoter P*_{lac}* by promoter P_{R} of the phage lambda and then treated with the *Bam*HI and *Sac*I restrictases. Thus, the plasmid pMW-P_{R}-pfkA containing the *pfkA* gene under the control of promoter P_{R} was constructed. Non-regulated high level of *pfkA* gene expression could be achieved using this plasmid.

The pMW-P_{R}-pfkA plasmid was introduced into the streptomycin-resistant threonine producer *E. coli* strain B-3996 (US patent 5,175,107). Thus, the strain B-3996(pMW-P_{R}-pfkA) was obtained.

Accumulation of L-threonine by *E. coli* strains B-3996 and B-3996(pMW-P_{R}-pfkA) was evaluated as described above (see Example 1). The results are presented in Table 2.

**Table 2.**

| Strain | OD₅₆₀ | Threonine, g/l |
|---|---|---|
| B-3996 | 9.7±0.1 | 14.3±0.1 |
| B-3996(pMW-P_{R}-pfkA) | 9.3±0.1 | 14.4±0.1 |

### Since the effect of enhanced expression of pfkA gene on L-threonine production in test tube fermentation was not impressive, the batch fermentation was performed in laboratory fermenters having a capacity of 1.0 liter.

For that purpose, the *E. coli* strains VKPM-3996 and VKPM-3996(pM-P_{R}-pfkA) were grown during 18-24 hours at 37 °C on L-agar plates containing streptomycin (100 µg/ml). Then one loop of the cells was transferred to 50 ml of L-broth of the following composition: tryptone - 10g/l, yeast extract - 5 g/l, NaCl - 5 g/l. The cells (50 ml, OD₅₄₀ - 2 o.u.) grown at 37 °C within 5 hours on shaker (240 rpm) was used for seeding 450 ml of the medium for fermentation. The batch fermentation was performed in laboratory fermenters having a capacity of 1.01 under aeration (1/1 vvm) with stirring at a speed of 1200 rpm at 37 °C. The pH value was maintained automatically at 6.6 using 8% ammonia liquor. The results are presented in Table 3.

The composition of the fermentation medium (g/l):

| | |
|---|---|
| Glucose | 100.0 |
| NH₄Cl | 1.75 |
| KH₂PO₄ | 1.0 |
| MgSO₄.7H₂O | 0.8 |
| FeSO₄.7H₂O | 0.01 |
| MnSO₄.5H₂O | 0.01 |
| Mameno(TN) | 0.15 |
| Betaine | 1.0 |
| L-isoleucine | 0.2 |

Glucose and magnesium sulfate are sterilized separately. pH is adjusted to 6.6.

**Table 3**

| Strain | OD₅₆₀ (final) | Threonine, g/l |
|---|---|---|
| B-3996 | 39.9±2.1 | 33.80±3.1 |
| B-3996(pMW-P_{R}-pfkA) | 34.4±1.5 | 39.57±1.0 |

As it is seen from the Table 3, the enhancement of *pfkA* gene expression improved L-threonine productivity of the strain B-3996.

### Example 3: Cloning of fbaA gene from E. coli and effect of enhanced expression of fbaA gene on L-threonine production.

The *fbaA* gene was obtained by PCR using chromosomal DNA of the *E*. *coli* strain MG 1655 (VKPM B-6195) as the template and primers P5 (SEQ ID NO: 21) and P6 (SEQ ID NO: 22). Primer P5 contains the recognition site of *Bam*HI restrictases introduced in the 5'-end thereof. Primer P6 contains recognition site of *Sac*I restrictases introduced in the 5'-end thereof. The obtained DNA fragment (1155 bp) containing *fbaA* gene was treated with *Bam*HI and *Sac*I restrictases and cloned into the plasmid pMW119 previously modified to substitute promoter P*_{lac}* by promoter P_{R} of the phage lambda and then treated with the same restrictases. Thus, the plasmid pMW-P_{R}-fbaA containing the *fbaA* gene under the control of promoter P_{R} was constructed. Non-regulated high level of *fbaA* gene expression could be achieved using this plasmid.

The pMW-P_{R}-fbaA plasmid was introduced into the streptomycin-resistant threonine producer *E. coli* strain B-3996 (US patent 5,175,107). Thus, the strain B-3996(pMW-P_{R}-fbaA) was obtained.

Accumulation of L-threonine by *E. coli* strains B-3996 and B-3996(pMW-P_{R}-fbaA) was evaluated as described above (see Example 1). The results are presented in Table 4.

**Table 4.**

| Strain | OD₅₆₀ | Threonine, g/l |
|---|---|---|
| B-3996 | 9.7±0.1 | 14.3±0.1 |
| B-3996(pMW-P_{R}-fbaA) | 9.3±0.2 | 15.1±0.5 |

As it is seen from the Table 4, the enhancement of *fbaA* gene expression improved L-threonine productivity of the strain B-3996.

### Example 4: Cloning of tpiA gene from E. coli and effect of enhanced expression of tpiA gene on L-threonine production.

The *tpiA* gene was obtained by PCR using chromosomal DNA of the *E*. *coli* strain MG 1655 (VKPM B-6195) as the template and primers P7 (SEQ ID NO: 23) and P8 (SEQ ID NO: 24). Primer P7 contains recognition site of *Bam*HI restrictases introduced in the 5'-end thereof. Primer P8 contains recognition site of *Sac*I restrictases introduced in the 5'-send thereof. The obtained DNA fragment (774 bp) containing *tpiA* gene was treated with *Bam*HI and *Sac*I restrictases and cloned into the plasmid pMW119 previously modified to substitute promoter *P_{lac}* by promoter P_{R} of the phage lambda and then treated with the same restrictases. Thus, the plasmid pMW-P_{R}-tpiA containing the *tpiA* gene under the control of promoter P_{R} was constructed. Non-regulated high levels of *tpiA* gene expression could be achieved using this plasmid.

The pMW-P_{R}-tpiA plasmid was introduced into the streptomycin-resistant threonine producer *E. coli* strain B-3996 (US patent 5,175,107). Thus, the strain B-3996(pMW-P_{R}-tpiA) was obtained.

Accumulation of L-threonine by *E. coli* strains B-3996 and B-3996(pMW-P_{R}-tpiA) was evaluated as described above (see Example 1). The results are presented in Table 5.

**Table 5.**

| Strain | OD₅₆₀ | Threonine, g/l |
|---|---|---|
| B-3996 | 9.7±0.1 | 14.3±0.1 |
| B-3996(pMW-P_{R}-tpiA) | 9.6±0.5 | 14.6±0.1 |

Since the effect of enhanced expression of *tpiA* gene on L-threonine production in test tube fermentation was not impressive, the batch fermentation was performed in laboratory fermenters having a capacity of 1.0 liter as described above (see Example 2). The results are presented in Table 6.

**Table 6.**

| Strain | OD₅₆₀ (final) | Threonine, g/l |
|---|---|---|
| B-3996 | 39.9±2.1 | 33.80±3.1 |
| B-3996(pMW-P_{R}-tpiA) | 34.9±2.6 | 37.16±1.4 |

As it is seen from the Table 6, the enhancement of *tpiA* gene expression improved L-threonine productivity of the strain B-3996.

### Example 5: Cloning of gapA gene from E. coli and effect of enhanced expression of gapA gene on L-threonine production.

To study the effect of enhanced expression of *gapA* gene on L-threonine production, *gapA* gene was cloned into plasmid pMW119 under control of mutant synthetic promoter (A3m) derived from A3 promoter of coliphage T3 (Yamada, M. et al. Promoter sequence analysis in Bacillus and Escherichia coli: construction of strong promoter in E. coli. Gene, 99(1), 109-114 (1991)). The mutations introduced into the A3 promoter sequence led to decreased promoter strength of this constitutive polymerase holoenzyme Es⁷⁰ - dependent promoter. Sequences of the oligonucleotides formed the mutant synthetic promoter A3m are shown in SEQ ID NO: 25 and 26. Structure of the promoter A3m is depicted on Fig. 1.

The *gapA* gene was obtained by PCR using chromosomal DNA of the *E. coli* strain MG 1655 (VKPM B-6195) as the template and primers gapA-5' (SEQ ID NO: 27) and gapA-ter (SEQ ID NO: 28). Primer gapA-5' contains recognition site of *Bam*HI restrictases introduced in the 5'-end thereof. Primer gapA-ter contains recognition site of *Xba*I restrictases introduced in the 5'-end thereof. Obtained DNA fragment (1.2 kbp) containing *gapA* gene with 5'-untranslated region up to P1 start transcription site and without own promoter's region was treated with *Bam*HI and *Xba*I restrictases, ligated with synthetic A3m promoter having sticky ends of *EcoRI* and *Bam*HI restriction sites and cloned into vector pMW119 previously treated with *EcoRI* and *XbaI* restrictases. Thus, the plasmid pMW119-PA3m-gapA was obtained. The structure of *gapA* gene was confirmed by sequencing.

*E. coli* cells HB101 were transformed with the plasmid pMW119-PA3m-gapA (Sambrook J. and Russell D.W. 2001. Molecular cloning: a laboratory manual, 3rd ed. Cold Spring Harbor Laboratory Press, Cold Spring Harbor, N.Y.). The strain MG1655 is available from American Type Culture Collection (ATCC33694). And activities of GAPDH at mid-log growth phase in strains HB101and HB101 (pMW-PA3m-gapA) were determined according the procedure described in by Peng, L., and Shimizu, K. (Appl. Microbiol. Biotechnol. 61, 163-178 (2003)). The results are presented in Table 7.

**Table 7.**

| Strain | Activity (nmol/mg ∗ min) |
|---|---|
| HB101 | 39 |
| HB101(pMW-PA3m-gapA) | 86 |

As it is seen from Table 7, the HB101 cells harboring the plasmid possessed more than 2-time higher GAPDH activity.

Then the pMW-PA3m-gapA plasmid was introduced into the streptomycin-resistant threonine producer *E. coli* strain B-3996 (US patent 5,175,107). Thus, the strain B-3996(pMW-PA3m-gapA) was obtained.

Both *E*. *coli* strains B-3996 and B-3996(pMW119-pA3-gapA) were grown overnight at 37 °C on LB-agar plates, or LB-agar plates containing ampicillin (100 µg/ml) in the case of the strain B-3996 (pMW119-pA3-gapA). One loop (OD595 - 2 - 3 o.u.) of the night cell culture of each strains was transferred to 2 ml of minimal test tube fermentation medium of the following composition: yeast extract - 2,0 g/l, (NH₄)₂SO₄ - 16.0 g/l, K₂HPO₄ - 0.7 g/l, MgSO₄•7H₂O - 1.0 g/l, MnSO₄•5H₂O - 0.01 g/l, FeSO₄•7H₂O - 0.01 g/l, thiamine HCl (B₁) - 0.2 mg/l, glucose - 4 %, CaCO₃ (chalk) - 30.0 g/l, L-isoleucine - 50 mg/l, ampicillin - 100 µg/ml (only in case of the strain 3996 (pMW119-pA3-gapA)). The cells were grown 48 h at 32 °C under permanent rotating (250 rpm).

After cultivation, the accumulated amount of L-threonine in the medium was determined by paper chromatography. The mobile phase has the following composition: n-butanol : acetic acid :water = 4 : 1 : 1. The amino acids were colored by ethanol solution of ninhydrine (1%) containing CdCl₂ (0,5%). After 1 hour incubation at 37 °C, the samples were measured at OD₅₀₈. The results are presented in Table 8.

**Table 8.**

| Strain | OD₅₅₅ | Threonine, g/l |
|---|---|---|
| B-3996 | 13.2±0.1 | 13.6±0.2 |
| B-3996(pMW-PA3m-gapA) | 13.3±0.1 | 15.6±0.3 |

As it is seen from the Table 8, the enhancement of *gapA* gene expression improved L-threonine productivity of the strain B-3996.

### Example 6: Cloning of eno gene from E. coli and effect of enhanced expression of eno gene on L-threonine production.

The *eno* gene was obtained by PCR using chromosomal DNA of the *E*. *coli* strain MG 1655 (VKPM B-6195) as the template and primers P9 (SEQ ID NO: 29) and P10 (SEQ ID NO: 30). Primer P9 contains recognition site of *Bam*HI restrictases introduced in the 5'-end thereof. Primer P10 contains recognition site of *Sac*I restrictases introduced in the 5'-end thereof. The obtained DNA fragment (1298 bp) containing the *eno* gene was treated with *Bam*HI and *Sac*I restrictases and cloned into the plasmid pMW119 previously modified to substitute promoter P*_{lac}* by promoter P_{R} of the phage lambda and then treated with the same restrictases. Thus, the plasmid pMW-P_{R}-eno containing the *eno* gene under the control of promoter P_{R} was constructed. Non-regulated high level of *eno* gene expression could be achieved using this plasmid.

The pMW-P_{R}-eno plasmid was introduced into the streptomycin-resistant threonine producer *E. coli* strain B-3996 (US patent 5,175,107). Thus, the strain B-3996(pMW-P_{R}-eno) was obtained.

Accumulation of L-threonine by *E. coli* strains B-3996 and B-3996(pMW-PR-eno) was evaluated as described above (see Example 1). The results are presented in Table 9.

**Table 9.**

| Strain | OD₅₆₀ | Threonine, g/l |
|---|---|---|
| B-3996 | 9.7±0.1 | 14.3±0.1 |
| B-3996(pMW-P_{R}-eno) | 9.3±0.2 | 14.9±0.4 |

As it is seen from the Table 9, the enhancement of eno gene expression improved L-threonine productivity of the strain B-3996.

### Example 7: Cloning of pgi gene from E. coli and effect of enhanced expression of pgi gene on L-threonine production:

The *pgi* gene was obtained by PCR using chromosomal DNA of the *E. coli* strain MG 1655 (VKPM B-6195) as the template and primers P11 (SEQ ID NO: 31) and P12 (SEQ ID NO: 32). Primer P11 contains recognition site of *Bam*HI restrictases introduced in the 5'-end thereof. Primer P12 contains recognition site of *Sac*I restrictases introduced in the 5'-end thereof. The obtained DNA fragment (1657 bp) containing *pgi* gene was treated with *Bam*HI and *Sac*I restrictases and cloned into the plasmid pMW119 previously modified to substitute promoter P*_{lac}* by promoter P_{R} of the phage lambda and then treated with the same restrictases. Thus, the plasmid pMW-P_{R}-pgi containing the *pgi* gene under the control of promoter P_{R} was constructed. Non-regulated high level of *pgi* gene expression could be achieved using this plasmid.

The pMW-P_{R}-pgi plasmid was introduced into the streptomycin-resistant threonine producer *E. coli* strain B-3996 (US patent 5,175,107). Thus, the strain B-3996(pMW-P_{R}-pgi) was obtained.

Accumulation of L-threonine by *E*. *coli* strains B-3996 and B-3996(pMW-P_{R}-pgi) was evaluated as described above (see Example 1). The results are presented in Table 10.

**Table 10.**

| Strain | OD₅₆₀ | Threonine, g/l |
|---|---|---|
| B-3996 | 8.7±0.4 | 18.4±0.7 |
| B-3996(pMW-P_{R}-pgi) | 8.4±0.2 | 19.7±0.4 |

As it is seen from the Table 10, the enhancement of *pgi* gene expression improved L-threonine productivity of the strain B-3996.

### Example 8: Cloning of pgk gene from E. coli and effect of enhanced expression of pgk gene on L-threonine production.

The *pgk* gene was obtained by PCR using chromosomal DNA of the *E. coli* strain MG 1655 (VKPM B-6195) as the template and primers P13 (SEQ ID NO: 33) and P14 (SEQ ID NO: 34). Primer P13 contains recognition site of *Bam*HI restrictases introduced in the 5'-end thereof. Primer P14 contains recognition site of *Sac*I restrictases introduced in the 5'-end thereof. The obtained DNA fragment (1163 bp) containing the *pgk* gene was treated with *Bam*HI and *Sac*I restrictases and cloned into the plasmid pMW119 previously modified to substitute promoter P*_{lac}* by promoter P_{R} of the phage lambda and then treated with the same restrictases. Thus, the plasmid pMW-P_{R}-pgk containing the *pgk* gene under the control of promoter P_{R} was constructed. Non-regulated high level of *pgk* gene expression could be achieved using this plasmid.

The pMW-P_{R}-pgi plasmid was introduced into the streptomycin-resistant threonine producer *E. coli* strain B-3996 (US patent 5,175,107). Thus, the strain B-3996(pMW-P_{R}-pgk) was obtained.

Accumulation of L-threonine by *E. coli* strains B-3996 and B-3996(pMW-P_{R}-pgk) was evaluated as described above (see Example 1). The results are presented in Table 11.

**Table 11:**

| Strain | OD₅₆₀ | Threonine, g/l |
|---|---|---|
| B-3996 | 10.3±0.5 | 19.2±0.2 |
| B-3996(pMW-P_{R}-pgk) | 10.2±0:5 | 20.3±0.4 |

As it is seen from the Table 11, the enhancement of *pgk* gene expression improved L-threonine productivity of the strain B-3996.

While the invention has been described in detail with reference to preferred embodiments thereof, it will be apparent to one skilled in the art that various changes can be made, and equivalents employed, without departing from the scope of the invention.

### Industrial Applicability

According to the present invention, production of L-threonine can be improved.

## Claims

1. A method for producing L-threonine, which comprises cultivating an L-threonine-producing *Escherichia coli* bacterium in a culture medium to produce and cause accumulation of L-threonine in the culture medium, and collecting the L-threonine from the culture medium, wherein the bacterium contains a modification by which the expression amount of one or more of the genes chosen from the group consisting of *glk, pgi, pfkA, tpiA, gapA, pgk, eno* and *pykA,* is enhanced over the parental strain by increasing the copy number of the gene or genes, or modifying an expression control sequence of the gene or genes so that the expression of the gene or genes is enhanced, and wherein the copy number is increased by transformation of the bacterium with a low copy vector containing the gene or genes.

2. The method according to claim 1, wherein the genes of claim 1 are originated from a bacterium belonging to the genus *Escherichia.*

3. The method according to any one of claims 1- 2, wherein the bacterium has been further modified to enhance expression of one or more genes selected from the group consisting of:
- the mutant *thrA* gene which codes for aspartokinase homoserine dehydrogenase I resistant to feed back inhibition by threonine;
- the *thrB* gene, which codes for homoserine kinase;
- the *thrC* gene, which codes for threonine synthase;
- the *rhtA* gene, which codes for a putative transmembrane protein.

4. The method according to claim 3, wherein the bacterium has been modified to increase expression amount of the mutant *thrA* gene, the *thrB* gene, the *thrC* gene and the *rhtA* gene.

5. The method according to any one of claims 1- 4, wherein the bacterium has been modified to enhance expression of the *glk* gene.

6. The method according to any one of claims 1- 4, wherein the bacterium has been modified to enhance expression of the *pgi* gene.

7. The method according to any one of claims 1- 4, wherein the bacterium has been modified to enhance expression of the *pfkA* gene.

8. The method according to any one of claims 1- 4, wherein the bacterium has been modified to enhance expression of the *tpiA* gene.

9. The method according to any one of claims 1- 4, wherein the bacterium has been modified to enhance expression of the *gapA* gene.

10. The method according to any one of claims 1- 4, wherein the bacterium has been modified to enhance expression of the *pgk* gene.

11. The method according to any one of claims 1- 4, wherein the bacterium has been modified to enhance expression of the *eno* gene.

12. The method according to any one of claims 1- 4, wherein the bacterium has been modified to enhance expression of the *pykA* gene.

## Patentansprüche

1. Verfahren zur Herstellung von L-Threonin, welches das Kultivieren eines L-Threonin-produzierenden Escherichia coli-Bakteriums in einem Kulturmedium, um L-Threonin herzustellen und eine Anreicherung von L-Threonin in dem Kulturmedium hervorzurufen, und Gewinnen von L-Threonin aus dem Kulturmedium umfasst, wobei das Bakterium eine Modifikation enthält, durch die die Expressionsmenge eines Gens oder mehrerer Gene, ausgewählt aus der Gruppe bestehend aus glk, pgi, pfkA, tpiA, gapA, pgk, eno und pykA, gegenüber dem Elternstamm verbessert wird, indem die Kopienzahl des Gens oder der Gene gesteigert wird oder eine Expressionskontrollsequenz des Gens oder der Gene modifiziert wird, so dass die Expression des Gens oder der Gene verbessert wird, und wobei die Kopienzahl durch Transformation des Bakteriums mit einem low copy-Vektor, der das Gen oder die Gene enthält, gesteigert wird.

2. Verfahren gemäß Anspruch 1, wobei sich die Gene gemäß Anspruch 1 von einem Bakterium ableiten, das zur Gattung Escherichia gehört.

3. Verfahren gemäß einem der Ansprüche 1 bis 2, wobei das Bakterium weiter modifiziert wurde, um die Expression eines Gens oder mehrerer Gene, ausgewählt aus der Gruppe bestehend aus:
- das mutierte thrA-Gen, das für die Aspartokinase-Homoserin-Dehydrogenase I kodiert, die gegenüber der Feedback-Hemmung durch Threonin resistent ist;
- das thrB-Gen, das für die Homoserinkinase kodiert;
- das thrC-Gen, das für die Threoninsynthase kodiert;
- das rhtA-Gen, das für ein putatives Transmembranprotein kodiert,
zu verbessern.

4. Verfahren gemäß Anspruch 3, wobei das Bakterium modifiziert wurde, um die Expressionsmenge des mutierten thrA-Gens, des thrB-Gens, des thrC-Gens und des rhtA-Gens zu steigern.

5. Verfahren gemäß einem der Ansprüche 1 bis 4, wobei das Bakterium modifiziert wurde, um die Expression des glk-Gens zu verbessern.

6. Verfahren gemäß einem der Ansprüche 1 bis 4, wobei das Bakterium modifiziert wurde, um die Expression des pgi-Gens zu verbessern.

7. Verfahren gemäß einem der Ansprüche 1 bis 4, wobei das Bakterium modifiziert wurde, um die Expression des pfkA-Gens zu verbessern.

8. Verfahren gemäß einem der Ansprüche 1 bis 4, wobei das Bakterium modifiziert wurde, um die Expression des tpiA-Gens zu verbessern.

9. Verfahren gemäß einem der Ansprüche 1 bis 4, wobei das Bakterium modifiziert wurde, um die Expression des gapA-Gens zu verbessern.

10. Verfahren gemäß einem der Ansprüche 1 bis 4, wobei das Bakterium modifiziert wurde, um die Expression des pgk-Gens zu verbessern.

11. Verfahren gemäß einem der Ansprüche 1 bis 4, wobei das Bakterium modifiziert wurde, um die Expression des eno-Gens zu verbessern.

12. Verfahren gemäß einem der Ansprüche 1 bis 4, wobei das Bakterium modifiziert wurde, um die Expression des pykA-Gens zu verbessern.

## Revendications

1. Procédé pour la production de L-thréonine, qui comprend la culture d'une bactérie *Escherichia coli* productrice de L-thréonine dans un milieu de culture pour produire et provoquer l'accumulation de L-thréonine dans le milieu de culture, et le recueil de la L-thréonine à partir du milieu de culture, dans lequel la bactérie contient une modification par laquelle la quantité d'expression d'un ou plusieurs des gènes choisis dans le groupe constitué par *glk, pgi, pfkA, tpiA, gapA, pgk, eno* et *pykA,* est améliorée par rapport à la souche parentale par l'augmentation du nombre de copies du gène ou des gènes, ou la modification d'une séquence de contrôle de l'expression du gène ou des gènes de sorte que l'expression du gène ou des gènes est améliorée, et dans lequel le nombre de copies est augmenté par transformation de la bactérie avec un vecteur à faible nombre de copies contenant le gène ou les gènes.

2. Procédé selon la revendication 1, dans lequel les gènes selon la revendication 1 sont issus d'une bactérie appartenant au genre *Escherichia.*

3. Procédé selon l'une quelconque des revendications 1 à 2, dans lequel la bactérie a en outre été modifiée pour améliorer l'expression d'un ou plusieurs gènes choisis dans le groupe constitué par :
- le gène *thrA* mutant qui code pour une aspartokinase homosérine déshydrogénase I résistante à l'inhibition par rétroaction par la thréonine ;
- le gène *thrB,* qui code pour une homosérine kinase ;
- le gène *thrC,* qui code pour une thréonine synthase ;
- le gène *rhtA,* qui code pour une protéine transmembranaire présumée.

4. Procédé selon la revendication 3, dans lequel la bactérie a été modifiée pour augmenter la quantité d'expression du gène *thrA* mutant, du gène *thrB,* du gène *thrC* et du gène *rhtA.*

5. Procédé selon l'une quelconque des revendications 1 à 4, dans lequel la bactérie a été modifiée pour améliorer l'expression du gène *glk.*

6. Procédé selon l'une quelconque des revendications 1 à 4, dans lequel la bactérie a été modifiée pour améliorer l'expression du gène *pgi.*

7. Procédé selon l'une quelconque des revendications 1 à 4, dans lequel la bactérie a été modifiée pour améliorer l'expression du gène *pfkA.*

8. Procédé selon l'une quelconque des revendications 1 à 4, dans lequel la bactérie a été modifiée pour améliorer l'expression du gène *tpiA.*

9. Procédé selon l'une quelconque des revendications 1 à 4, dans lequel la bactérie a été modifiée pour améliorer l'expression du gène *gapA.*

10. Procédé selon l'une quelconque des revendications 1 à 4, dans lequel la bactérie a été modifiée pour améliorer l'expression du gène *pgk.*

11. Procédé selon l'une quelconque des revendications 1 à 4, dans lequel la bactérie a été modifiée pour améliorer l'expression du gène *eno.*

12. Procédé selon l'une quelconque des revendications 1 à 4, dans lequel la bactérie a été modifiée pour améliorer l'expression du gène *pykA.*


### SEQUENCE LISTING

<110> Ajinomoto Co., Inc.
<120> METHOD FOR PRODUCING L-THREONINE USING BACTERIA
   BELONGING TO THE GENUS ESCHERICHIA
<130> C3440PC4332
<150> RU2004103986
   <151> 2004-02-12
<150> US60/601144
   <151> 2004-08-13
<160> 34
<170> PatentIn Ver. 2.1
<210> 1
   <211> 966
   <212> DNA
   <213> Escherichia coli
<220>
   <221> CDS
   <222> (1)..(966)
<400> 1
   atg aca aag tat gca tta gtc ggt gat gtg ggc ggc acc aac gca cgt      48
   Met Thr Lys Tyr Ala Leu Val Gly Asp Val Gly Gly Thr Asn Ala Arg
   1               5                   10                  15
   ctt gct ctg tgt gat att gcc agt ggt gaa atc tcg cag gct aag acc      96
   Leu Ala Leu Cys Asp Ile Ala Ser Gly Glu Ile Ser Gln Ala Lys Thr
               20                  25                  30
   tat tca ggg ctt gat tac ccc agc ctc gaa gcg gtc att cgc gtt tat     144
   Tyr Ser Gly Leu Asp Tyr Pro Ser Leu Glu Ala Val Ile Arg Val Tyr
           35                  40                  45
   ctt gaa gaa cat aag gtc gag gtg aaa gac ggc tgt att gcc atc gct     192
   Leu Glu Glu His Lys Val Glu Val Lys Asp Gly Cys Ile Ala Ile Ala
       50                  55                  60
   tgc cca att acc ggt gac tgg gtg gcg atg acc aac cat acc tgg gcg     240
   Cys Pro Ile Thr Gly Asp Trp Val Ala Met Thr Asn His Thr Trp Ala
   65                  70                  75                  80
   ttc tca att gcc gaa atg aaa aag aat ctc ggt ttt agc cat ctg gaa     288
   Phe Ser Ile Ala Glu Met Lys Lys Asn Leu Gly Phe Ser His Leu Glu
                   85                  90                  95
   att att aac gat ttt acc gct gta tcg atg gcg atc ccg atg ctg aaa     336
   Ile Ile Asn Asp Phe Thr Ala Val Ser Met Ala Ile Pro Met Leu Lys
               100                 105                 110
   aaa gag cat ctg att cag ttt ggt ggc gca gaa ccg gtc gaa ggt aag     384
   Lys Glu His Leu Ile Gln Phe Gly Gly Ala Glu Pro Val Glu Gly Lys
           115                 120                 125
   cct att gcg gtt tac ggt gcc gga acg ggg ctt ggg gtt gcg cat ctg      432
   Pro Ile Ala Val Tyr Gly Ala Gly Thr Gly Leu Gly Val Ala His Leu
       130                 135                 140
   gtc cat gtc gat aag cgt tgg gta agc ttg cca ggc gaa ggc ggt cac      480
   Val His Val Asp Lys Arg Trp Val Ser Leu Pro Gly Glu Gly Gly His
   145                 150                 155                 160
   gtt gat ttt gcg ccg aat agt gaa gaa gag gcc att atc ctc gaa ata      528
   Val Asp Phe Ala Pro Asn Ser Glu Glu Glu Ala Ile Ile Leu Glu Ile
                   165                 170                 175
   ttg cgt gcg gaa att ggt cat gtt tcg gcg gag cgc gtg ctt tct ggc      576
   Leu Arg Ala Glu Ile Gly His Val Ser Ala Glu Arg Val Leu Ser Gly
               180                 185                 190
   cct ggg ctg gtg aat ttg tat cgc gca att gtg aaa gct gac aac cgc      624
   Pro Gly Leu Val Asn Leu Tyr Arg Ala Ile Val Lys Ala Asp Asn Arg
           195                 200                 205
   ctg cca gaa aat ctc aag cca aaa gat att acc gaa cgc gcg ctg gct      672
   Leu Pro Glu Asn Leu Lys Pro Lys Asp Ile Thr Glu Arg Ala Leu Ala
       210                 215                 220
   gac agc tgc acc gat tgc cgc cgc gca ttg tcg ctg ttt tgc gtc att      720
   Asp Ser Cys Thr Asp Cys Arg Arg Ala Leu Ser Leu Phe Cys Val Ile
   225                 230                 235                 240
   atg ggc cgt ttt ggc ggc aat ctg gcg ctc aat ctc ggg aca ttt ggc      768
   Met Gly Arg Phe Gly Gly Asn Leu Ala Leu Asn Leu Gly Thr Phe Gly
                   245                 250                 255
   ggc gtg ttt att gcg ggc ggt atc gtg ccg cgc ttc ctt gag ttc ttc      816
   Gly Val Phe Ile Ala Gly Gly Ile Val Pro Arg Phe Leu Glu Phe Phe
               260                 265                 270
   aaa gcc tcc ggt ttc cgt gcc gca ttt gaa gat aaa ggg cgc ttt aaa      864
   Lys Ala Ser Gly Phe Arg Ala Ala Phe Glu Asp Lys Gly Arg Phe Lys
           275                 280                 285
   gaa tat gtc cat gat att ccg gtg tat ctc atc gtc cat gac aat ccg      912
   Glu Tyr Val His Asp Ile Pro Val Tyr Leu Ile Val His Asp Asn Pro
       290                 295                 300
   ggc ctt ctc ggt tcc ggt gca cat tta cgc cag acc tta ggt cac att      960
   Gly Leu Leu Gly Ser Gly Ala His Leu Arg Gln Thr Leu Gly His Ile
   305                 310                 315                 320
   ctg taa                                                              966
   Leu
<210> 2
   <211> 321
   <212> PRT
   <213> Escherichia coli
<400> 2
   Met Thr Lys Tyr Ala Leu Val Gly Asp Val Gly Gly Thr Asn Ala Arg
   1               5                   10                  15
   Leu Ala Leu Cys Asp Ile Ala Ser Gly Glu Ile Ser Gln Ala Lys Thr
               20                  25                  30
   Tyr Ser Gly Leu Asp Tyr Pro Ser Leu Glu Ala Val Ile Arg Val Tyr
           35                  40                  45
   Leu Glu Glu His Lys Val Glu Val Lys Asp Gly Cys Ile Ala Ile Ala
       50                  55                  60
   Cys Pro Ile Thr Gly Asp Trp Val Ala Met Thr Asn His Thr Trp Ala
   65                  70                  75                  80
   Phe Ser Ile Ala Glu Met Lys Lys Asn Leu Gly Phe Ser His Leu Glu
                   85                  90                  95
   Ile Ile Asn Asp Phe Thr Ala Val Ser Met Ala Ile Pro Met Leu Lys
               100                 105                 110
   Lys Glu His Leu Ile Gln Phe Gly Gly Ala Glu Pro Val Glu Gly Lys
           115                 120                 125
   Pro Ile Ala Val Tyr Gly Ala Gly Thr Gly Leu Gly Val Ala His Leu
       130                 135                 140
   Val His Val Asp Lys Arg Trp Val Ser Leu Pro Gly Glu Gly Gly His
   145                 150                 155                 160
   Val Asp Phe Ala Pro Asn Ser Glu Glu Glu Ala Ile Ile Leu Glu Ile
                   165                 170                 175
   Leu Arg Ala Glu Ile Gly His Val Ser Ala Glu Arg Val Leu Ser Gly
               180                 185                 190
   Pro Gly Leu Val Asn Leu Tyr Arg Ala Ile Val Lys Ala Asp Asn Arg
           195                 200                 205
   Leu Pro Glu Asn Leu Lys Pro Lys Asp Ile Thr Glu Arg Ala Leu Ala
       210                 215                 220
   Asp Ser Cys Thr Asp Cys Arg Arg Ala Leu Ser Leu Phe Cys Val Ile
   225                 230                 235                 240
   Met Gly Arg Phe Gly Gly Asn Leu Ala Leu Asn Leu Gly Thr Phe Gly
                   245                 250                 255
   Gly Val Phe Ile Ala Gly Gly Ile Val Pro Arg Phe Leu Glu Phe Phe
               260                 265                 270
   Lys Ala Ser Gly Phe Arg Ala Ala Phe Glu Asp Lys Gly Arg Phe Lys
           275                 280                 285
   Glu Tyr Val His Asp Ile Pro Val Tyr Leu Ile Val His Asp Asn Pro
       290                 295                 300
   Gly Leu Leu Gly Ser Gly Ala His Leu Arg Gln Thr Leu Gly His Ile
   305                 310                 315                 320
   Leu
<210> 3
   <211> 1650
   <212> DNA
   <213> Escherichia coli
<220>
   <221> CDS
   <222> (1)..(1650)
<400> 3
   atg aaa aac atc aat cca acg cag acc gct gcc tgg cag gca cta cag    48
   Met Lys Asn Ile Asn Pro Thr Gln Thr Ala Ala Trp Gln Ala Leu Gln
    1               5                  10                  15
   aaa cac ttc gat gaa atg aaa gac gtt acg atc gcc gat ctt ttt gct    96
   Lys His Phe Asp Glu Met Lys Asp Val Thr Ile Ala Asp Leu Phe Ala
               20                  25                  30
   aaa gac ggc gat cgt ttt tct aag ttc tcc gca acc ttc gac gat cag     144
   Lys Asp Gly Asp Arg Phe Ser Lys Phe Ser Ala Thr Phe Asp Asp Gln
           35                  40                  45
   atg ctg gtg gat tac tcc aaa aac cgc atc act gaa gag acg ctg gcg     192
   Met Leu Val Asp Tyr Ser Lys Asn Arg Ile Thr Glu Glu Thr Leu Ala
       50                  55                  60
   aaa tta cag gat ctg gcg aaa gag tgc gat ctg gcg ggc gcg att aag     240
   Lys Leu Gln Asp Leu Ala Lys Glu Cys Asp Leu Ala Gly Ala Ile Lys
   65                  70                  75                  80
   tcg atg ttc tct ggc gag aag atc aac cgc act gaa aac cgc gcc gtg     288
   Ser Met Phe Ser Gly Glu Lys Ile Asn Arg Thr Glu Asn Arg Ala Val
                   85                  90                  95
   ctg cac gta gcg ctg cgt aac cgt agc aat acc ccg att ttg gtt gat     336
   Leu His Val Ala Leu Arg Asn Arg Ser Asn Thr Pro Ile Leu Val Asp
               100                 105                 110
   ggc aaa gac gta atg ccg gaa gtc aac gcg gtg ctg gag aag atg aaa     384
   Gly Lys Asp Val Met Pro Glu Val Asn Ala Val Leu Glu Lys Met Lys
           115                 120                 125
   acc ttc tca gaa gcg att att tcc ggt gag tgg aaa ggt tat acc ggc     432
   Thr Phe Ser Glu Ala Ile Ile Ser Gly Glu Trp Lys Gly Tyr Thr Gly
       130                 135                 140
   aaa gca atc act gac gta gtg aac atc ggg atc ggc ggt tct gac ctc     480
   Lys Ala Ile Thr Asp Val Val Asn Ile Gly Ile Gly Gly Ser Asp Leu
   145                 150                 155                 160
   ggc cca tac atg gtg acc gaa gct ctg cgt ccg tac aaa aac cac ctg     528
   Gly Pro Tyr Met Val Thr Glu Ala Leu Arg Pro Tyr Lys Asn His Leu
                   165                 170                 175
   aac atg cac ttt gtt tct aac gtc gat ggg act cac atc gcg gaa gtg     576
   Asn Met His Phe Val Ser Asn Val Asp Gly Thr His Ile Ala Glu Val
               180                 185                 190
   ctg aaa aaa gta aac ccg gaa acc acg ctg ttc ttg gta gca tct aaa     624
   Leu Lys Lys Val Asn Pro Glu Thr Thr Leu Phe Leu Val Ala Ser Lys
           195                 200                 205
   acc ttc acc act cag gaa act atg acc aac gcc cat agc gcg cgt gac     672
   Thr Phe Thr Thr Gln Glu Thr Met Thr Asn Ala His Ser Ala Arg Asp
       210                 215                 220
   tgg ttc ctg aaa gcg gca ggt gat gaa aaa cac gtt gca aaa cac ttt     720
   Trp Phe Leu Lys Ala Ala Gly Asp Glu Lys His Val Ala Lys His Phe
   225                 230                 235                 240
   gcg gcg ctt tcc acc aat gcc aaa gcc gtt ggc gag ttt ggt att gat     768
   Ala Ala Leu Ser Thr Asn Ala Lys Ala Val Gly Glu Phe Gly Ile Asp
                   245                 250                 255
   act gcc aac atg ttc gag ttc tgg gac tgg gtt ggc ggc cgt tac tct     816
   Thr Ala Asn Met Phe Glu Phe Trp Asp Trp Val Gly Gly Arg Tyr Ser
               260                 265                 270
   ttg tgg tca gcg att ggc ctg tcg att gtt ctc tcc atc ggc ttt gat     864
   Leu Trp Ser Ala Ile Gly Leu Ser Ile Val Leu Ser Ile Gly Phe Asp
           275                 280                 285
   aac ttc gtt gaa ctg ctt tcc ggc gca cac gcg atg gac aag cat ttc     912
   Asn Phe Val Glu Leu Leu Ser Gly Ala His Ala Met Asp Lys His Phe
       290                 295                 300
   tcc acc acg cct gcc gag aaa aac ctg cct gta ctg ctg gcg ctg att     960
   Ser Thr Thr Pro Ala Glu Lys Asn Leu Pro Val Leu Leu Ala Leu Ile
   305                 310                 315                 320
   ggc atc tgg tac aac aat ttc ttt ggt gcg gaa act gaa gcg att ctg    1008
   Gly Ile Trp Tyr Asn Asn Phe Phe Gly Ala Glu Thr Glu Ala Ile Leu
                   325                 330                 335
   ccg tat gac cag tat atg cac cgt ttc gcg gcg tac ttc cag cag ggc    1056
   Pro Tyr Asp Gln Tyr Met His Arg Phe Ala Ala Tyr Phe Gln Gln Gly
               340                 345                 350
   aat atg gag tcc aac ggt aag tat gtt gac cgt aac ggt aac gtt gtg    1104
   Asn Met Glu Ser Asn Gly Lys Tyr Val Asp Arg Asn Gly Asn Val Val
           355                 360                 365
   gat tac cag act ggc ccg att atc tgg ggt gaa cca ggc act aac ggt    1152
   Asp Tyr Gln Thr Gly Pro Ile Ile Trp Gly Glu Pro Gly Thr Asn Gly
       370                 375                 380
   cag cac gcg ttc tac cag ctg atc cac cag gga acc aaa atg gta ccg    1200
   Gln His Ala Phe Tyr Gln Leu Ile His Gln Gly Thr Lys Met Val Pro
   385                 390                 395                 400
   tgc gat ttc atc gct ccg gct atc acc cat aac ccg ctc tct gat cat    1248
   Cys Asp Phe Ile Ala Pro Ala Ile Thr His Asn Pro Leu Ser Asp His
                   405                 410                 415
   cac cag aaa ctg ctg tct aac ttc ttc gcc cag acc gaa gcg ctg gcg    1296
   His Gln Lys Leu Leu Ser Asn Phe Phe Ala Gln Thr Glu Ala Leu Ala
               420                 425                 430
   ttt ggt aaa tcc cgc gaa gtg gtt gag cag gaa tat cgt gat cag ggt    1344
   Phe Gly Lys Ser Arg Glu Val Val Glu Gln Glu Tyr Arg Asp Gln Gly
           435                 440                 445
   aaa gat ccg gca acg ctt gac tac gtg gtg ccg ttc aaa gta ttc gaa    1392
   Lys Asp Pro Ala Thr Leu Asp Tyr Val Val Pro Phe Lys Val Phe Glu
       450                 455                 460
   ggt aac cgc ccg acc aac tcc atc ctg ctg cgt gaa atc act ccg ttc    1440
   Gly Asn Arg Pro Thr Asn Ser Ile Leu Leu Arg Glu Ile Thr Pro Phe
   465                 470                 475                 480
   agc ctg ggt gcg ttg att gcg ctg tat gag cac aaa atc ttt act cag    1488
   Ser Leu Gly Ala Leu Ile Ala Leu Tyr Glu His Lys Ile Phe Thr Gln
                   485                 490                 495
   ggc gtg atc ctg aac atc ttc acc ttc gac cag tgg ggc gtg gaa ctg    1536
   Gly Val Ile Leu Asn Ile Phe Thr Phe Asp Gln Trp Gly Val Glu Leu
               500                 505                 510
   ggt aaa cag ctg gcg aac cgt att ctg cca gag ctg aaa gat gat aaa    1584
   Gly Lys Gln Leu Ala Asn Arg Ile Leu Pro Glu Leu Lys Asp Asp Lys
           515                 520                 525
   gaa atc agc agc cac gat agc tcg acc aat ggt ctg att aac cgc tat    1632
   Glu Ile Ser Ser His Asp Ser Ser Thr Asn Gly Leu Ile Asn Arg Tyr
       530                 535                 540
   aaa gcg tgg cgc ggt taa                                            1650
   Lys Ala Trp Arg Gly
   545                 550
<210> 4
   <211> 549
   <212> PRT
   <213> Escherichia coli
<400> 4
   Met Lys Asn Ile Asn Pro Thr Gln Thr Ala Ala Trp Gln Ala Leu Gln
   1               5                   10                  15
   Lys His Phe Asp Glu Met Lys Asp Val Thr Ile Ala Asp Leu Phe Ala
               20                  25                  30
   Lys Asp Gly Asp Arg Phe Ser Lys Phe Ser Ala Thr Phe Asp Asp Gln
           35                  40                  45
   Met Leu Val Asp Tyr Ser Lys Asn Arg Ile Thr Glu Glu Thr Leu Ala
       50                  55                  60
   Lys Leu Gln Asp Leu Ala Lys Glu Cys Asp Leu Ala Gly Ala Ile Lys
   65                  70                  75                  80
   Ser Met Phe Ser Gly Glu Lys Ile Asn Arg Thr Glu Asn Arg Ala Val
                   85                  90                  95
   Leu His Val Ala Leu Arg Asn Arg Ser Asn Thr Pro Ile Leu Val Asp
               100                 105                 110
   Gly Lys Asp Val Met Pro Glu Val Asn Ala Val Leu Glu Lys Met Lys
           115                 120                 125
   Thr Phe Ser Glu Ala Ile Ile Ser Gly Glu Trp Lys Gly Tyr Thr Gly
       130                 135                 140
   Lys Ala Ile Thr Asp Val Val Asn Ile Gly Ile Gly Gly Ser Asp Leu
   145                 150                 155                 160
   Gly Pro Tyr Met Val Thr Glu Ala Leu Arg Pro Tyr Lys Asn His Leu
                   165                 170                 175
   Asn Met His Phe Val Ser Asn Val Asp Gly Thr His Ile Ala Glu Val
               180                 185                 190
   Leu Lys Lys Val Asn Pro Glu Thr Thr Leu Phe Leu Val Ala Ser Lys
           195                 200                 205
   Thr Phe Thr Thr Gln Glu Thr Met Thr Asn Ala His Ser Ala Arg Asp
       210                 215                 220
   Trp Phe Leu Lys Ala Ala Gly Asp Glu Lys His Val Ala Lys His Phe
   225                 230                 235                 240
   Ala Ala Leu Ser Thr Asn Ala Lys Ala Val Gly Glu Phe Gly Ile Asp
                   245                 250                 255
   Thr Ala Asn Met Phe Glu Phe Trp Asp Trp Val Gly Gly Arg Tyr Ser
               260                 265                 270
   Leu Trp Ser Ala Ile Gly Leu Ser Ile Val Leu Ser Ile Gly Phe Asp
           275                 280                 285
   Asn Phe Val Glu Leu Leu Ser Gly Ala His Ala Met Asp Lys His Phe
       290                 295                 300
   Ser Thr Thr Pro Ala Glu Lys Asn Leu Pro Val Leu Leu Ala Leu Ile
   305                 310                 315                 320
   Gly Ile Trp Tyr Asn Asn Phe Phe Gly Ala Glu Thr Glu Ala Ile Leu
                   325                 330                 335
   Pro Tyr Asp Gln Tyr Met His Arg Phe Ala Ala Tyr Phe Gln Gln Gly
               340                 345                 350
   Asn Met Glu Ser Asn Gly Lys Tyr Val Asp Arg Asn Gly Asn Val Val
           355                 360                 365
   Asp Tyr Gln Thr Gly Pro Ile Ile Trp Gly Glu Pro Gly Thr Asn Gly
       370                 375                 380
   Gln His Ala Phe Tyr Gln Leu Ile His Gln Gly Thr Lys Met Val Pro
   385                 390                 395                 400
   Cys Asp Phe Ile Ala Pro Ala Ile Thr His Asn Pro Leu Ser Asp His
                   405                 410                 415
   His Gln Lys Leu Leu Ser Asn Phe Phe Ala Gln Thr Glu Ala Leu Ala
               420                 425                 430
   Phe Gly Lys Ser Arg Glu Val Val Glu Gln Glu Tyr Arg Asp Gln Gly
           435                 440                 445
   Lys Asp Pro Ala Thr Leu Asp Tyr Val Val Pro Phe Lys Val Phe Glu
       450                 455                 460
   Gly Asn Arg Pro Thr Asn Ser Ile Leu Leu Arg Glu Ile Thr Pro Phe
   465                 470                 475                 480
   Ser Leu Gly Ala Leu Ile Ala Leu Tyr Glu His Lys Ile Phe Thr Gln
                   485                 490                 495
   Gly Val Ile Leu Asn Ile Phe Thr Phe Asp Gln Trp Gly Val Glu Leu
               500                 505                 510
   Gly Lys Gln Leu Ala Asn Arg Ile Leu Pro Glu Leu Lys Asp Asp Lys
           515                 520                 525
   Glu Ile Ser Ser His Asp Ser Ser Thr Asn Gly Leu Ile Asn Arg Tyr
       530                 535                 540
   Lys Ala Trp Arg Gly
   545
<210> 5
   <211> 963
   <212> DNA
   <213> Escherichia coli
<220>
   <221> CDS
   <222> (1)..(963)
<400> 5
   atg att aag aaa atc ggt gtg ttg aca agc ggc ggt gat gcg cca ggc      48
   Met Ile Lys Lys Ile Gly Val Leu Thr Ser Gly Gly Asp Ala Pro Gly
   1               5                   10                  15
   atg aac gcc gca att cgc ggg gtt gtt cgt tct gcg ctg aca gaa ggt      96
   Met Asn Ala Ala Ile Arg Gly Val Val Arg Ser Ala Leu Thr Glu Gly
               20                  25                  30
   ctg gaa gta atg ggt att tat gac ggc tat ctg ggt ctg tat gaa gac     144
   Leu Glu Val Met Gly Ile Tyr Asp Gly Tyr Leu Gly Leu Tyr Glu Asp
           35                  40                  45
   cgt atg gta cag cta gac cgt tac agc gtg tct gac atg atc aac cgt     192
   Arg Met Val Gln Leu Asp Arg Tyr Ser Val Ser Asp Met Ile Asn Arg
       50                  55                  60
   ggc ggt acg ttc ctc ggt tct gcg cgt ttc ccg gaa ttc cgc gac gag     240
   Gly Gly Thr Phe Leu Gly Ser Ala Arg Phe Pro Glu Phe Arg Asp Glu
    65                  70                  75                  80
   aac atc cgc gcc gtg gct atc gaa aac ctg aaa aaa cgt ggt atc gac     288
   Asn Ile Arg Ala Val Ala Ile Glu Asn Leu Lys Lys Arg Gly Ile Asp
                    85                  90                  95
   gcg ctg gtg gtt atc ggc ggt gac ggt tcc tac atg ggt gca atg cgt     336
   Ala Leu Val Val Ile Gly Gly Asp Gly Ser Tyr Met Gly Ala Met Arg
               100                 105                 110
   ctg acc gaa atg ggc ttc ccg tgc atc ggt ctg ccg ggc act atc gac     384
   Leu Thr Glu Met Gly Phe Pro Cys Ile Gly Leu Pro Gly Thr Ile Asp
           115                 120                 125
   aac gac atc aaa ggc act gac tac act atc ggt ttc ttc act gcg ctg     432
   Asn Asp Ile Lys Gly Thr Asp Tyr Thr Ile Gly Phe Phe Thr Ala Leu
       130                 135                 140
   agc acc gtt gta gaa gcg atc gac cgt ctg cgt gac acc tct tct tct     480
   Ser Thr Val Val Glu Ala Ile Asp Arg Leu Arg Asp Thr Ser Ser Ser
   145                 150                 155                 160
   cac cag cgt att tcc gtg gtg gaa gtg atg ggc cgt tat tgt gga gat     528
   His Gln Arg Ile Ser Val Val Glu Val Met Gly Arg Tyr Cys Gly Asp
                   165                 170                 175
   ctg acg ttg gct gcg gcc att gcc ggt ggc tgt gaa ttc gtt gtg gtt     576
   Leu Thr Leu Ala Ala Ala Ile Ala Gly Gly Cys Glu Phe Val Val Val
               180                 185                 190
   ccg gaa gtt gaa ttc agc cgt gaa gac ctg gta aac gaa atc aaa gcg     624
   Pro Glu Val Glu Phe Ser Arg Glu Asp Leu Val Asn Glu Ile Lys Ala
           195                 200                 205
   ggt atc gcg aaa ggt aaa aaa cac gcg atc gtg gcg att acc gaa cat     672
   Gly Ile Ala Lys Gly Lys Lys His Ala Ile Val Ala Ile Thr Glu His
       210                 215                 220
   atg tgt gat gtt gac gaa ctg gcg cat ttc atc gag aaa gaa acc ggt     720
   Met Cys Asp Val Asp Glu Leu Ala His Phe Ile Glu Lys Glu Thr Gly
   225                 230                 235                 240
   cgt gaa acc cgc gca act gtg ctg ggc cac atc cag cgc ggt ggt tct     768
   Arg Glu Thr Arg Ala Thr Val Leu Gly His Ile Gln Arg Gly Gly Ser
                   245                 250                 255
   ccg gtg cct tac gac cgt att ctg gct tcc cgt atg ggc gct tac gct     816
   Pro Val Pro Tyr Asp Arg Ile Leu Ala Ser Arg Met Gly Ala Tyr Ala
               260                 265                 270
   atc gat ctg ctg ctg gca ggt tac ggc ggt cgt tgt gta ggt atc cag     864
   Ile Asp Leu Leu Leu Ala Gly Tyr Gly Gly Arg Cys Val Gly Ile Gln
           275                 280                 285
   aac gaa cag ctg gtt cac cac gac atc atc gac gct atc gaa aac atg     912
   Asn Glu Gln Leu Val His His Asp Ile Ile Asp Ala Ile Glu Asn Met
       290                 295                 300
   aag cgt ccg ttc aaa ggt gac tgg ctg gac tgc gcg aaa aaa ctg tat     960
   Lys Arg Pro Phe Lys Gly Asp Trp Leu Asp Cys Ala Lys Lys Leu Tyr
   305                 310                 315                 320
   taa                                                                   963
<210> 6
   <211> 320
   <212> PRT
   <213> Escherichia coli
<400> 6
   Met Ile Lys Lys Ile Gly Val Leu Thr Ser Gly Gly Asp Ala Pro Gly
    1               5                   10                  15
   Met Asn Ala Ala Ile Arg Gly Val Val Arg Ser Ala Leu Thr Glu Gly
               20                  25                  30
   Leu Glu Val Met Gly Ile Tyr Asp Gly Tyr Leu Gly Leu Tyr Glu Asp
           35                  40                  45
   Arg Met Val Gln Leu Asp Arg Tyr Ser Val Ser Asp Met Ile Asn Arg
       50                  55                  60
   Gly Gly Thr Phe Leu Gly Ser Ala Arg Phe Pro Glu Phe Arg Asp Glu
   65                  70                  75                  80
   Asn Ile Arg Ala Val Ala Ile Glu Asn Leu Lys Lys Arg Gly Ile Asp
                   85                  90                  95
   Ala Leu Val Val Ile Gly Gly Asp Gly Ser Tyr Met Gly Ala Met Arg
               100                 105                 110
   Leu Thr Glu Met Gly Phe Pro Cys Ile Gly Leu Pro Gly Thr Ile Asp
           115                 120                 125
   Asn Asp Ile Lys Gly Thr Asp Tyr Thr Ile Gly Phe Phe Thr Ala Leu
       130                 135                 140
   Ser Thr Val Val Glu Ala Ile Asp Arg Leu Arg Asp Thr Ser Ser Ser
   145                 150                 155                 160
   His Gln Arg Ile Ser Val Val Glu Val Met Gly Arg Tyr Cys Gly Asp
                   165                 170                 175
   Leu Thr Leu Ala Ala Ala Ile Ala Gly Gly Cys Glu Phe Val Val Val
               180                 185                 190
   Pro Glu Val Glu Phe Ser Arg Glu Asp Leu Val Asn Glu Ile Lys Ala
           195                 200                 205
   Gly Ile Ala Lys Gly Lys Lys His Ala Ile Val Ala Ile Thr Glu His
       210                 215                 220
   Met Cys Asp Val Asp Glu Leu Ala His Phe Ile Glu Lys Glu Thr Gly
   225                 230                 235                 240
   Arg Glu Thr Arg Ala Thr Val Leu Gly His Ile Gln Arg Gly Gly Ser
                   245                 250                 255
   Pro Val Pro Tyr Asp Arg Ile Leu Ala Ser Arg Met Gly Ala Tyr Ala
               260                 265                 270
   Ile Asp Leu Leu Leu Ala Gly Tyr Gly Gly Arg Cys Val Gly Ile Gln
           275                 280                 285
   Asn Glu Gln Leu Val His His Asp Ile Ile Asp Ala Ile Glu Asn Met
       290                 295                 300
   Lys Arg Pro Phe Lys Gly Asp Trp Leu Asp Cys Ala Lys Lys Leu Tyr
   305                 310                 315                 320
<210> 7
   <211> 768
   <212> DNA
   <213> Escherichia coli
<220>
   <221> CDS
   <222> (1)..(768)
<400> 7
   atg cga cat cct tta gtg atg ggt aac tgg aaa ctg aac ggc agc cgc      48
   Met Arg His Pro Leu Val Met Gly Asn Trp Lys Leu Asn Gly Ser Arg
   1               5                   10                  15
   cac atg gtt cac gag ctg gtt tct aac ctg cgt aaa gag ctg gca ggt      96
   His Met Val His Glu Leu Val Ser Asn Leu Arg Lys Glu Leu Ala Gly
               20                  25                  30
   gtt gct ggc tgt gcg gtt gca atc gca cca ccg gaa atg tat atc gat      144
   Val Ala Gly Cys Ala Val Ala Ile Ala Pro Pro Glu Met Tyr Ile Asp
           35                  40                  45
   atg gcg aag cgc gaa gct gaa ggc agc cac atc atg ctg ggt gcg caa      192
   Met Ala Lys Arg Glu Ala Glu Gly Ser His Ile Met Leu Gly Ala Gln
       50                  55                  60
   aac gtg gac ctg aac ctg tcc ggc gca ttc acc ggt gaa acc tct gct      240
   Asn Val Asp Leu Asn Leu Ser Gly Ala Phe Thr Gly Glu Thr Ser Ala
   65                  70                  75                  80
   gct atg ctg aaa gac atc ggc gca cag tac atc atc atc ggt cac tct      288
   Ala Met Leu Lys Asp Ile Gly Ala Gln Tyr Ile Ile Ile Gly His Ser
                   85                  90                  95
   gaa cgt cgt act tac cac aaa gaa tct gac gaa ctg atc gcg aaa aaa      336
   Glu Arg Arg Thr Tyr His Lys Glu Ser Asp Glu Leu Ile Ala Lys Lys
               100                 105                 110
   ttc gcg gtg ctg aaa gag cag ggc ctg act ccg gtt ctg tgc atc ggt      384
   Phe Ala Val Leu Lys Glu Gln Gly Leu Thr Pro Val Leu Cys Ile Gly
           115                 120                 125
   gaa acc gaa gct gaa aat gaa gcg ggc aaa act gaa gaa gtt tgc gca      432
   Glu Thr Glu Ala Glu Asn Glu Ala Gly Lys Thr Glu Glu Val Cys Ala
       130                 135                 140
   cgt cag atc gac gcg gta ctg aaa act cag ggt gct gcg gca ttc gaa      480
   Arg Gln Ile Asp Ala Val Leu Lys Thr Gln Gly Ala Ala Ala Phe Glu
   145                 150                 155                 160
   ggt gcg gtt atc gct tac gaa cct gta tgg gca atc ggt act ggc aaa      528
   Gly Ala Val Ile Ala Tyr Glu Pro Val Trp Ala Ile Gly Thr Gly Lys
                   165                 170                 175
   tct gca act ccg gct cag gca cag gct gtt cac aaa ttc atc cgt gac      576
   Ser Ala Thr Pro Ala Gln Ala Gln Ala Val His Lys Phe Ile Arg Asp
               180                 185                 190
   cac atc gct aaa gtt gac gct aac atc gct gaa caa gtg atc att cag      624
   His Ile Ala Lys Val Asp Ala Asn Ile Ala Glu Gln Val Ile Ile Gln
           195                 200                 205
   tac ggc ggc tct gta aac gcg tct aac gct gca gaa ctg ttt gct cag      672
   Tyr Gly Gly Ser Val Asn Ala Ser Asn Ala Ala Glu Leu Phe Ala Gln
           210                 215                 220
   ccg gat atc gac ggc gcg ctg gtt ggt ggt gct tct ctg aaa gct gac    720
   Pro Asp Ile Asp Gly Ala Leu Val Gly Gly Ala Ser Leu Lys Ala Asp
   225                 230                 235                 240
   gcc ttc gca gta atc gtt aaa gct gca gaa gcg gct aaa cag gct taa    768
   Ala Phe Ala Val Ile Val Lys Ala Ala Glu Ala Ala Lys Gln Ala
                   245                 250                 255
<210> 8
   <211> 255
   <212> PRT
   <213> Escherichia coli
<400> 8
   Met Arg His Pro Leu Val Met Gly Asn Trp Lys Leu Asn Gly Ser Arg
   1               5                   10                  15
   His Met Val His Glu Leu Val Ser Asn Leu Arg Lys Glu Leu Ala Gly
               20                  25                  30
   Val Ala Gly Cys Ala Val Ala Ile Ala Pro Pro Glu Met Tyr Ile Asp
           35                  40                  45
   Met Ala Lys Arg Glu Ala Glu Gly Ser His Ile Met Leu Gly Ala Gln
       50                  55                  60
   Asn Val Asp Leu Asn Leu Ser Gly Ala Phe Thr Gly Glu Thr Ser Ala
   65                  70                  75                  80
   Ala Met Leu Lys Asp Ile Gly Ala Gln Tyr Ile Ile Ile Gly His Ser
                   85                  90                  95
   Glu Arg Arg Thr Tyr His Lys Glu Ser Asp Glu Leu Ile Ala Lys Lys
               100                 105                 110
   Phe Ala Val Leu Lys Glu Gln Gly Leu Thr Pro Val Leu Cys Ile Gly
           115                 120                 125
   Glu Thr Glu Ala Glu Asn Glu Ala Gly Lys Thr Glu Glu Val Cys Ala
       130                 135                 140
   Arg Gln Ile Asp Ala Val Leu Lys Thr Gln Gly Ala Ala Ala Phe Glu
   145                 150                 155                 160
   Gly Ala Val Ile Ala Tyr Glu Pro Val Trp Ala Ile Gly Thr Gly Lys
                   165                 170                 175
   Ser Ala Thr Pro Ala Gln Ala Gln Ala Val His Lys Phe Ile Arg Asp
               180                 185                 190
   His Ile Ala Lys Val Asp Ala Asn Ile Ala Glu Gln Val Ile Ile Gln
           195                 200                 205
   Tyr Gly Gly Ser Val Asn Ala Ser Asn Ala Ala Glu Leu Phe Ala Gln
       210                 215                 220
   Pro Asp Ile Asp Gly Ala Leu Val Gly Gly Ala Ser Leu Lys Ala Asp
   225                 230                 235                 240
   Ala Phe Ala Val Ile Val Lys Ala Ala Glu Ala Ala Lys Gln Ala
                   245                 250                 255
<210> 9
   <211> 996
   <212> DNA
   <213> Escherichia coli
<220>
   <221> CDS
   <222> (1)..(996)
<400> 9
   atg act atc aaa gta ggt atc aac ggt ttt ggc cgt atc ggt cgc att    48
   Met Thr Ile Lys Val Gly Ile Asn Gly Phe Gly Arg Ile Gly Arg Ile
   1               5                   10                  15
   gtt ttc cgt gct gct cag aaa cgt tct gac atc gag atc gtt gca atc    96
   Val Phe Arg Ala Ala Gln Lys Arg Ser Asp Ile Glu Ile Val Ala Ile
               20                  25                  30
   aac gac ctg tta gac gct gat tac atg gca tac atg ctg aaa tat gac    144
   Asn Asp Leu Leu Asp Ala Asp Tyr Met Ala Tyr Met Leu Lys Tyr Asp
           35                  40                  45
   tcc act cac ggc cgt ttc gac ggt acc gtt gaa gtg aaa gac ggt cat    192
   Ser Thr His Gly Arg Phe Asp Gly Thr Val Glu Val Lys Asp Gly His
       50                  55                  60
   ctg atc gtt aac ggt aaa aaa atc cgt gtt acc gct gaa cgt gat ccg    240
   Leu Ile Val Asn Gly Lys Lys Ile Arg Val Thr Ala Glu Arg Asp Pro
   65                  70                  75                  80
   gct aac ctg aaa tgg gac gaa gtt ggt gtt gac gtt gtc gct gaa gca    288
   Ala Asn Leu Lys Trp Asp Glu Val Gly Val Asp Val Val Ala Glu Ala
                   85                  90                  95
   act ggt ctg ttc ctg act gac gaa act gct cgt aaa cac atc acc gct    336
   Thr Gly Leu Phe Leu Thr Asp Glu Thr Ala Arg Lys His Ile Thr Ala
               100                 105                 110
   ggt gcg aag aaa gtg gtt atg act ggt ccg tct aaa gac aac act ccg    384
   Gly Ala Lys Lys Val Val Met Thr Gly Pro Ser Lys Asp Asn Thr Pro
           115                 120                 125
   atg ttc gtt aaa ggc gct aac ttc gac aaa tat gct ggc cag gac atc    432
   Met Phe Val Lys Gly Ala Asn Phe Asp Lys Tyr Ala Gly Gln Asp Ile
       130                 135                 140
   gtt tcc aac gct tcc tgc acc acc aac tgc ctg gct ccg ctg gct aaa    480
   Val Ser Asn Ala Ser Cys Thr Thr Asn Cys Leu Ala Pro Leu Ala Lys
   145                 150                 155                 160
   gtt atc aac gat aac ttc ggc atc atc gaa ggt ctg atg acc acc gtt    528
   Val Ile Asn Asp Asn Phe Gly Ile Ile Glu Gly Leu Met Thr Thr Val
                   165                 170                 175
   cac gct act acc gct act cag aaa acc gtt gat ggc ccg tct cac aaa    576
   His Ala Thr Thr Ala Thr Gln Lys Thr Val Asp Gly Pro Ser His Lys
               180                 185                 190
   gac tgg cgc ggc ggc cgc ggc gct tcc cag aac atc atc ccg tcc tct    624
   Asp Trp Arg Gly Gly Arg Gly Ala Ser Gln Asn Ile Ile Pro Ser Ser
           195                 200                 205
   acc ggt gct gct aaa gct gta ggt aaa gta ctg cca gaa ctg aat ggc    672
   Thr Gly Ala Ala Lys Ala Val Gly Lys Val Leu Pro Glu Leu Asn Gly
       210                 215                 220
   aaa ctg act ggt atg gcg ttc cgc gtt ccg acc ccg aac gta tct gta    720
   Lys Leu Thr Gly Met Ala Phe Arg Val Pro Thr Pro Asn Val Ser Val
   225                 230                 235                 240
   gtt gac ctg acc gtt cgt ctg gaa aaa gct gca act tac gag cag atc     768
   Val Asp Leu Thr Val Arg Leu Glu Lys Ala Ala Thr Tyr Glu Gln Ile
                   245                 250                 255
   aaa gct gcc gtt aaa gct gct gct gaa ggc gaa atg aaa ggc gtt ctg     816
   Lys Ala Ala Val Lys Ala Ala Ala Glu Gly Glu Met Lys Gly Val Leu
               260                 265                 270
   ggc tac acc gaa gat gac gta gta tct acc gat ttc aac ggc gaa gtt     864
   Gly Tyr Thr Glu Asp Asp Val Val Ser Thr Asp Phe Asn Gly Glu Val
           275                 280                 285
   tgc act tcc gtg ttc gat gct aaa gct ggt atc gct ctg aac gac aac     912
   Cys Thr Ser Val Phe Asp Ala Lys Ala Gly Ile Ala Leu Asn Asp Asn
       290                 295                 300
   ttc gtg aaa ctg gta tcc tgg tac gac aac gaa acc ggt tac tcc aac     960
   Phe Val Lys Leu Val Ser Trp Tyr Asp Asn Glu Thr Gly Tyr Ser Asn
   305                 310                 315                 320
   aaa gtt ctg gac ctg atc gct cac atc tcc aaa taa                     996
   Lys Val Leu Asp Leu Ile Ala His Ile Ser Lys
                   325                 330
<210> 10
   <211> 331
   <212> PRT
   <213> Escherichia coli
<400> 10
   Met Thr Ile Lys Val Gly Ile Asn Gly Phe Gly Arg Ile Gly Arg Ile
   1 5 10 15
   Val Phe Arg Ala Ala Gln Lys Arg Ser Asp Ile Glu Ile Val Ala Ile
   20 25 30
   Asn Asp Leu Leu Asp Ala Asp Tyr Met Ala Tyr Met Leu Lys Tyr Asp
   35 40 45
   Ser Thr His Gly Arg Phe Asp Gly Thr Val Glu Val Lys Asp Gly His
   50 55 60
   Leu Ile Val Asn Gly Lys Lys Ile Arg Val Thr Ala Glu Arg Asp Pro
   65 70 75 80
   Ala Asn Leu Lys Trp Asp Glu Val Gly Val Asp Val Val Ala Glu Ala
   85 90 95
   Thr Gly Leu Phe Leu Thr Asp Glu Thr Ala Arg Lys His Ile Thr Ala
   100 105 110
   Gly Ala Lys Lys Val Val Met Thr Gly Pro Ser Lys Asp Asn Thr Pro
   115 120 125
   Met Phe Val Lys Gly Ala Asn Phe Asp Lys Tyr Ala Gly Gln Asp Ile
   130 135 140
   Val Ser Asn Ala Ser Cys Thr Thr Asn Cys Leu Ala Pro Leu Ala Lys
   145 150 155 160
   Val Ile Asn Asp Asn Phe Gly Ile Ile Glu Gly Leu Met Thr Thr Val
   165 170 175
   His Ala Thr Thr Ala Thr Gln Lys Thr Val Asp Gly Pro Ser His Lys
   180 185 190
   Asp Trp Arg Gly Gly Arg Gly Ala Ser Gln Asn Ile Ile Pro Ser Ser
   195 200 205
   Thr Gly Ala Ala Lys Ala Val Gly Lys Val Leu Pro Glu Leu Asn Gly
   210 215 220
   Lys Leu Thr Gly Met Ala Phe Arg Val Pro Thr Pro Asn Val Ser Val
   225 230 235 240
   Val Asp Leu Thr Val Arg Leu Glu Lys Ala Ala Thr Tyr Glu Gln Ile
   245 250 255
   Lys Ala Ala Val Lys Ala Ala Ala Glu Gly Glu Met Lys Gly Val Leu
   260 265 270
   Gly Tyr Thr Glu Asp Asp Val Val Ser Thr Asp Phe Asn Gly Glu Val
   275 280 285
   Cys Thr Ser Val Phe Asp Ala Lys Ala Gly Ile Ala Leu Asn Asp Asn
   290 295 300
   Phe Val Lys Leu Val Ser Trp Tyr Asp Asn Glu Thr Gly Tyr Ser Asn
   305 310 315 320
   Lys Val Leu Asp Leu Ile Ala His Ile Ser Lys
   325 330
<210> 11
   <211> 1164
   <212> DNA
   <213> Escherichia coli
<220>
   <221> CDS
   <222> (1)..(1164)
<400> 11
   atg tct gta att aag atg acc gat ctg gat ctt gct ggg aaa cgt gta   48
   Met Ser Val Ile Lys Met Thr Asp Leu Asp Leu Ala Gly Lys Arg Val
    1               5                  10                  15
   ttt atc cgt gcg gat ctg aac gta cca gta aaa gac ggg aaa gta acc   96
   Phe Ile Arg Ala Asp Leu Asn Val Pro Val Lys Asp Gly Lys Val Thr
               20                  25                  30
   agc gac gcg cgt atc cgt gct tct ctg ccg acc atc gaa ctg gcc ctg   144
   Ser Asp Ala Arg Ile Arg Ala Ser Leu Pro Thr Ile Glu Leu Ala Leu
           35                  40                  45
   aaa caa ggc gca aaa gtg atg gta act tcc cac ctg ggt cgt cct acc   192
   Lys Gln Gly Ala Lys Val Met Val Thr Ser His Leu Gly Arg Pro Thr
       50                  55                  60
   gaa ggc gag tac aac gaa gaa ttc tct ctg ctg ccg gtt gtt aac tac   240
   Glu Gly Glu Tyr Asn Glu Glu Phe Ser Leu Leu Pro Val Val Asn Tyr
   65                  70                  75                  80
   ctg aaa gac aaa ctg tct aac ccg gtt cgt ctg gtt aaa gat tac ctc   288
   Leu Lys Asp Lys Leu Ser Asn Pro Val Arg Leu Val Lys Asp Tyr Leu
                   85                  90                  95
   gac ggc gtt gac gtt gct gaa ggt gaa ctg gtt gtt ctg gaa aac gtt   336
   Asp Gly Val Asp Val Ala Glu Gly Glu Leu Val Val Leu Glu Asn Val
               100                 105                 110
   cgc ttc aac aaa ggc gag aag aaa gac gac gaa acc ctg tcc aaa aaa   384
   Arg Phe Asn Lys Gly Glu Lys Lys Asp Asp Glu Thr Leu Ser Lys Lys
           115                 120                 125
   tac gct gca ctg tgt gac gtg ttc gta atg gac gca ttc ggt act gct     432
   Tyr Ala Ala Leu Cys Asp Val Phe Val Met Asp Ala Phe Gly Thr Ala
       130                 135                 140
   cac cgc gcg cag gct tct act cac ggt atc ggt aaa ttc gct gac gtt     480
   His Arg Ala Gln Ala Ser Thr His Gly Ile Gly Lys Phe Ala Asp Val
   145                 150                 155                 160
   gcg tgc gca ggc ccg ctg ctg gca gct gaa ctg gac gcg ctg ggt aaa     528
   Ala Cys Ala Gly Pro Leu Leu Ala Ala Glu Leu Asp Ala Leu Gly Lys
                   165                 170                 175
   gca ctg aaa gaa cct gct cgc ccg atg gtg gct atc gtt ggt ggt tct     576
   Ala Leu Lys Glu Pro Ala Arg Pro Met Val Ala Ile Val Gly Gly Ser
               180                 185                 190
   aaa gta tct acc aaa ctg acc gtt ctg gac tcc ctg tct aaa atc gct     624
   Lys Val Ser Thr Lys Leu Thr Val Leu Asp Ser Leu Ser Lys Ile Ala
           195                 200                 205
   gac cag ctg att gtt ggt ggt ggt atc gct aac acc ttt atc gcg gca     672
   Asp Gln Leu Ile Val Gly Gly Gly Ile Ala Asn Thr Phe Ile Ala Ala
       210                 215                 220
   caa ggc cac gat gtg ggt aaa tcc ctg tac gaa gct gac ctg gtt gac     720
   Gln Gly His Asp Val Gly Lys Ser Leu Tyr Glu Ala Asp Leu Val Asp
   225                 230                 235                 240
   gaa gct aaa cgt ctg ctg acc acc tgc aac atc ccg gtt ccg tct gat     768
   Glu Ala Lys Arg Leu Leu Thr Thr Cys Asn Ile Pro Val Pro Ser Asp
                   245                 250                 255
   gtt cgc gta gca acc gag ttc tct gaa act gca ccg gct acc ctg aaa     816
   Val Arg Val Ala Thr Glu Phe Ser Glu Thr Ala Pro Ala Thr Leu Lys
               260                 265                 270
   tct gtt aac gat gtg aaa gct gac gag cag atc ctg gat atc ggt gat     864
   Ser Val Asn Asp Val Lys Ala Asp Glu Gln Ile Leu Asp Ile Gly Asp
           275                 280                 285
   gct tcc gct cag gaa ctg gct gaa atc ctg aag aat gcg aaa acc att     912
   Ala Ser Ala Gln Glu Leu Ala Glu Ile Leu Lys Asn Ala Lys Thr Ile
       290                 295                 300
   ctg tgg aac ggt ccg gtt ggc gtg ttc gaa ttc ccg aac ttc cgc aaa     960
   Leu Trp Asn Gly Pro Val Gly Val Phe Glu Phe Pro Asn Phe Arg Lys
   305                 310                 315                 320
   ggt act gaa atc gtg gct aac gct atc gca gac agc gaa gcg ttc tcc     1008
   Gly Thr Glu Ile Val Ala Asn Ala Ile Ala Asp Ser Glu Ala Phe Ser
                   325                 330                 335
   atc gct ggc ggc ggc gac act ctg gca gca atc gac ctg ttc ggc att     1056
   Ile Ala Gly Gly Gly Asp Thr Leu Ala Ala Ile Asp Leu Phe Gly Ile
               340                 345                 350
   gct gac aaa atc tcc tac atc tcc act ggc ggc ggc gca ttc ctc gaa     1104
   Ala Asp Lys Ile Ser Tyr Ile Ser Thr Gly Gly Gly Ala Phe Leu Glu
           355                 360                 365
   ttc gtg gaa ggt aaa gta ctg cct gca gta gcg atg ctc gaa gag cgc    1152
   Phe Val Glu Gly Lys Val Leu Pro Ala Val Ala Met Leu Glu Glu Arg
       370                 375                 380
   gct aag aag taa                                                    1164
   Ala Lys Lys
   385
<210> 12
   <211> 387
   <212> PRT
   <213> Escherichia coli
<400> 12
   Met Ser Val Ile Lys Met Thr Asp Leu Asp Leu Ala Gly Lys Arg Val
    1               5                  10                  15
   Phe Ile Arg Ala Asp Leu Asn Val Pro Val Lys Asp Gly Lys Val Thr
               20                  25                  30
   Ser Asp Ala Arg Ile Arg Ala Ser Leu Pro Thr Ile Glu Leu Ala Leu
           35                  40                  45
   Lys Gln Gly Ala Lys Val Met Val Thr Ser His Leu Gly Arg Pro Thr
       50                  55                  60
   Glu Gly Glu Tyr Asn Glu Glu Phe Ser Leu Leu Pro Val Val Asn Tyr
   65                  70                  75                  80
   Leu Lys Asp Lys Leu Ser Asn Pro Val Arg Leu Val Lys Asp Tyr Leu
                   85                  90                  95
   Asp Gly Val Asp Val Ala Glu Gly Glu Leu Val Val Leu Glu Asn Val
               100                 105                 110
   Arg Phe Asn Lys Gly Glu Lys Lys Asp Asp Glu Thr Leu Ser Lys Lys
           115                 120                 125
   Tyr Ala Ala Leu Cys Asp Val Phe Val Met Asp Ala Phe Gly Thr Ala
       130                 135                 140
   His Arg Ala Gln Ala Ser Thr His Gly Ile Gly Lys Phe Ala Asp Val
   145                 150                 155                 160
   Ala Cys Ala Gly Pro Leu Leu Ala Ala Glu Leu Asp Ala Leu Gly Lys
                   165                 170                 175
   Ala Leu Lys Glu Pro Ala Arg Pro Met Val Ala Ile Val Gly Gly Ser
               180                 185                 190
   Lys Val Ser Thr Lys Leu Thr Val Leu Asp Ser Leu Ser Lys Ile Ala
           195                 200                 205
   Asp Gln Leu Ile Val Gly Gly Gly Ile Ala Asn Thr Phe Ile Ala Ala
       210                 215                 220
   Gln Gly His Asp Val Gly Lys Ser Leu Tyr Glu Ala Asp Leu Val Asp
   225                 230                 235                 240
   Glu Ala Lys Arg Leu Leu Thr Thr Cys Asn Ile Pro Val Pro Ser Asp
                   245                 250                 255
   Val Arg Val Ala Thr Glu Phe Ser Glu Thr Ala Pro Ala Thr Leu Lys
               260                 265                 270
   Ser Val Asn Asp Val Lys Ala Asp Glu Gln Ile Leu Asp Ile Gly Asp
           275                 280                 285
   Ala Ser Ala Gln Glu Leu Ala Glu Ile Leu Lys Asn Ala Lys Thr Ile
       290                 295                 300
   Leu Trp Asn Gly Pro Val Gly Val Phe Glu Phe Pro Asn Phe Arg Lys
   305                 310                 315                 320
   Gly Thr Glu Ile Val Ala Asn Ala Ile Ala Asp Ser Glu Ala Phe Ser
                   325                 330                 335
   Ile Ala Gly Gly Gly Asp Thr Leu Ala Ala Ile Asp Leu Phe Gly Ile
               340                 345                 350
   Ala Asp Lys Ile Ser Tyr Ile Ser Thr Gly Gly Gly Ala Phe Leu Glu
           355                 360                 365
   Phe Val Glu Gly Lys Val Leu Pro Ala Val Ala Met Leu Glu Glu Arg
       370                 375                 380
   Ala Lys Lys
   385
<210> 13
   <211> 1299
   <212> DNA
   <213> Escherichia coli
<220>
   <221> CDS
   <222> (1)..(1299)
<400> 13
   atg tcc aaa atc gta aaa atc atc ggt cgt gaa atc atc gac tcc cgt      48
   Met Ser Lys Ile Val Lys Ile Ile Gly Arg Glu Ile Ile Asp Ser Arg
   1               5                   10                  15
   ggt aac ccg act gtt gaa gcc gaa gta cat ctg gag ggt ggt ttc gtc      96
   Gly Asn Pro Thr Val Glu Ala Glu Val His Leu Glu Gly Gly Phe Val
               20                  25                  30
   ggt atg gca gct gct ccg tca ggt gct tct act ggt tcc cgt gaa gct      144
   Gly Met Ala Ala Ala Pro Ser Gly Ala Ser Thr Gly Ser Arg Glu Ala
           35                  40                  45
   ctg gaa ctg cgc gat ggc gac aaa tcc cgt ttc ctg ggt aaa ggc gta      192
   Leu Glu Leu Arg Asp Gly Asp Lys Ser Arg Phe Leu Gly Lys Gly Val
       50                  55                  60
   acc aaa gct gtt gct gcg gta aac ggc ccg atc gct cag gcg ctg att      240
   Thr Lys Ala Val Ala Ala Val Asn Gly Pro Ile Ala Gln Ala Leu Ile
   65                  70                  75                  80
   ggc aaa gat gct aaa gat cag gct ggc att gac aag atc atg atc gac      288
   Gly Lys Asp Ala Lys Asp Gln Ala Gly Ile Asp Lys Ile Met Ile Asp
                   85                  90                  95
   ctg gac ggc acc gaa aac aaa tcc aaa ttc ggc gcg aac gca atc ctg      336
   Leu Asp Gly Thr Glu Asn Lys Ser Lys Phe Gly Ala Asn Ala Ile Leu
               100                 105                 110
   gct gta tct ctg gct aac gcc aaa gct gct gca gct gct aaa ggt atg      384
   Ala Val Ser Leu Ala Asn Ala Lys Ala Ala Ala Ala Ala Lys Gly Met
           115                 120                 125
   ccg ctg tac gag cac atc gct gaa ctg aac ggt act ccg ggc aaa tac      432
   Pro Leu Tyr Glu His Ile Ala Glu Leu Asn Gly Thr Pro Gly Lys Tyr
       130                 135                 140
   tct atg ccg gtt ccg atg atg aac atc atc aac ggt ggt gag cac gct      480
   Ser Met Pro Val Pro Met Met Asn Ile Ile Asn Gly Gly Glu His Ala
   145                 150                 155                 160
   gac aac aac gtt gat atc cag gaa ttc atg att cag ccg gtt ggc gcg      528
   Asp Asn Asn Val Asp Ile Gln Glu Phe Met Ile Gln Pro Val Gly Ala
               165                 170                 175
   aaa act gtg aaa gaa gcc atc cgc atg ggt tct gaa gtt ttc cat cac     576
   Lys Thr Val Lys Glu Ala Ile Arg Met Gly Ser Glu Val Phe His His
           180                 185                 190
   ctg gca aaa gtt ctg aaa gcg aaa ggc atg aac act gct gtt ggt gac     624
   Leu Ala Lys Val Leu Lys Ala Lys Gly Met Asn Thr Ala Val Gly Asp
       195                 200                 205
   gaa ggt ggc tat gcg ccg aac ctg ggt tcc aac gct gaa gct ctg gct     672
   Glu Gly Gly Tyr Ala Pro Asn Leu Gly Ser Asn Ala Glu Ala Leu Ala
       210                 215                 220
   gtt atc gct gaa gct gtt aaa gct gct ggt tat gaa ctg ggc aaa gac     720
   Val Ile Ala Glu Ala Val Lys Ala Ala Gly Tyr Glu Leu Gly Lys Asp
   225                 230                 235                 240
   atc act ttg gcg atg gac tgc gca gct tct gaa ttc tac aaa gat ggt     768
   Ile Thr Leu Ala Met Asp Cys Ala Ala Ser Glu Phe Tyr Lys Asp Gly
                   245                 250                 255
   aaa tac gtt ctg gct ggc gaa ggc aac aaa gcg ttc acc tct gaa gaa     816
   Lys Tyr Val Leu Ala Gly Glu Gly Asn Lys Ala Phe Thr Ser Glu Glu
               260                 265                 270
   ttc act cac ttc ctg gaa gaa ctg acc aaa cag tac ccg atc gtt tct     864
   Phe Thr His Phe Leu Glu Glu Leu Thr Lys Gln Tyr Pro Ile Val Ser
           275                 280                 285
   atc gaa gac ggt ctg gac gaa tct gac tgg gac ggt ttc gca tac cag     912
   Ile Glu Asp Gly Leu Asp Glu Ser Asp Trp Asp Gly Phe Ala Tyr Gln
       290                 295                 300
   acc aaa gtt ctg ggc gac aaa atc cag ctg gtt ggt gac gac ctg ttc     960
   Thr Lys Val Leu Gly Asp Lys Ile Gln Leu Val Gly Asp Asp Leu Phe
   305                 310                 315                 320
   gta acc aac acc aag atc ctg aaa gaa ggt atc gaa aaa ggt atc gct    1008
   Val Thr Asn Thr Lys Ile Leu Lys Glu Gly Ile Glu Lys Gly Ile Ala
                   325                 330                 335
   aac tcc atc ctg atc aaa ttc aac cag atc ggt tct ctg acc gaa act    1056
   Asn Ser Ile Leu Ile Lys Phe Asn Gln Ile Gly Ser Leu Thr Glu Thr
               340                 345                 350
   ctg gct gca atc aag atg gcg aaa gat gct ggc tac act gca gtt atc    1104
   Leu Ala Ala Ile Lys Met Ala Lys Asp Ala Gly Tyr Thr Ala Val Ile
           355                 360                 365
   tct cac cgt tct ggc gaa act gaa gac gct acc atc gct gac ctg gct    1152
   Ser His Arg Ser Gly Glu Thr Glu Asp Ala Thr Ile Ala Asp Leu Ala
       370                 375                 380
   gtt ggt act gct gca ggc cag atc aaa act ggt tct atg agc cgt tct    1200
   Val Gly Thr Ala Ala Gly Gln Ile Lys Thr Gly Ser Met Ser Arg Ser
   385                 390                 395                 400
   gac cgt gtt gct aaa tac aac cag ctg att cgt atc gaa gaa gct ctg    1248
   Asp Arg Val Ala Lys Tyr Asn Gln Leu Ile Arg Ile Glu Glu Ala Leu
   405 410 415
   ggc gaa aaa gca ccg tac aac ggt cgt aaa gag atc aaa ggc cag gca 1296
   Gly Glu Lys Ala Pro Tyr Asn Gly Arg Lys Glu Ile Lys Gly Gln Ala
   420 425 430
   taa 1299
<210> 14
   <211> 432
   <212> PRT
   <213> Escherichia coli
<400> 14
   Met Ser Lys Ile Val Lys Ile Ile Gly Arg Glu Ile Ile Asp Ser Arg
   1 5 10 15
   Gly Asn Pro Thr Val Glu Ala Glu Val His Leu Glu Gly Gly Phe Val
   20 25 30
   Gly Met Ala Ala Ala Pro Ser Gly Ala Ser Thr Gly Ser Arg Glu Ala
   35 40 45
   Leu Glu Leu Arg Asp Gly Asp Lys Ser Arg Phe Leu Gly Lys Gly Val
   50 55 60
   Thr Lys Ala Val Ala Ala Val Asn Gly Pro Ile Ala Gln Ala Leu Ile
   65 70 75 80
   Gly Lys Asp Ala Lys Asp Gln Ala Gly Ile Asp Lys Ile Met Ile Asp
   85 90 95
   Leu Asp Gly Thr Glu Asn Lys Ser Lys Phe Gly Ala Asn Ala Ile Leu
   100 105 110
   Ala Val Ser Leu Ala Asn Ala Lys Ala Ala Ala Ala Ala Lys Gly Met
   115 120 125
   Pro Leu Tyr Glu His Ile Ala Glu Leu Asn Gly Thr Pro Gly Lys Tyr
   130 135 140
   Ser Met Pro Val Pro Met Met Asn Ile Ile Asn Gly Gly Glu His Ala
   145 150 155 160
   Asp Asn Asn Val Asp Ile Gln Glu Phe Met Ile Gln Pro Val Gly Ala
   165 170 175
   Lys Thr Val Lys Glu Ala Ile Arg Met Gly Ser Glu Val Phe His His
   180 185 190
   Leu Ala Lys Val Leu Lys Ala Lys Gly Met Asn Thr Ala Val Gly Asp
   195 200 205
   Glu Gly Gly Tyr Ala Pro Asn Leu Gly Ser Asn Ala Glu Ala Leu Ala
   210 215 220
   Val Ile Ala Glu Ala Val Lys Ala Ala Gly Tyr Glu Leu Gly Lys Asp
   225 230 235 240
   Ile Thr Leu Ala Met Asp Cys Ala Ala Ser Glu Phe Tyr Lys Asp Gly
   245 250 255
   Lys Tyr Val Leu Ala Gly Glu Gly Asn Lys Ala Phe Thr Ser Glu Glu
   260 265 270
   Phe Thr His Phe Leu Glu Glu Leu Thr Lys Gln Tyr Pro Ile Val Ser
   275 280 285
   Ile Glu Asp Gly Leu Asp Glu Ser Asp Trp Asp Gly Phe Ala Tyr Gln
   290 295 300
   Thr Lys Val Leu Gly Asp Lys Ile Gln Leu Val Gly Asp Asp Leu Phe
   305 310 315 320
   Val Thr Asn Thr Lys Ile Leu Lys Glu Gly Ile Glu Lys Gly Ile Ala
   325 330 335
   Asn Ser Ile Leu Ile Lys Phe Asn Gln Ile Gly Ser Leu Thr Glu Thr
   340 345 350
   Leu Ala Ala Ile Lys Met Ala Lys Asp Ala Gly Tyr Thr Ala Val Ile
           355                 360                 365
   Ser His Arg Ser Gly Glu Thr Glu Asp Ala Thr Ile Ala Asp Leu Ala
       370                 375                 380
   Val Gly Thr Ala Ala Gly Gln Ile Lys Thr Gly Ser Met Ser Arg Ser
   385                 390                 395                 400
   Asp Arg Val Ala Lys Tyr Asn Gln Leu Ile Arg Ile Glu Glu Ala Leu
                   405                 410                 415
   Gly Glu Lys Ala Pro Tyr Asn Gly Arg Lys Glu Ile Lys Gly Gln Ala
               420                 425                 430
<210> 15
   <211> 1443
   <212> DNA
   <213> Escherichia coli
<220>
   <221> CDS
   <222> (1)..(1443)
<400> 15
   atg tcc aga agg ctt cgc aga aca aaa atc gtt acc acg tta ggc cca      48
   Met Ser Arg Arg Leu Arg Arg Thr Lys Ile Val Thr Thr Leu Gly Pro
   1               5                   10                  15
   gca aca gat cgc gat aat aat ctt gaa aaa gtt atc gcg gcg ggt gcc      96
   Ala Thr Asp Arg Asp Asn Asn Leu Glu Lys Val Ile Ala Ala Gly Ala
               20                  25                  30
   aac gtt gta cgt atg aac ttt tct cac ggc tcg cct gaa gat cac aaa     144
   Asn Val Val Arg Met Asn Phe Ser His Gly Ser Pro Glu Asp His Lys
           35                  40                  45
   atg cgc gcg gat aaa gtt cgt gag att gcc gca aaa ctg ggg cgt cat     192
   Met Arg Ala Asp Lys Val Arg Glu Ile Ala Ala Lys Leu Gly Arg His
       50                  55                  60
   gtg gct att ctg ggt gac ctc cag ggg ccc aaa atc cgt gta tcc acc     240
   Val Ala Ile Leu Gly Asp Leu Gln Gly Pro Lys Ile Arg Val Ser Thr
   65                  70                  75                  80
   ttt aaa gaa ggc aaa gtt ttc ctc aat att ggg gat aaa ttc ctg ctc     288
   Phe Lys Glu Gly Lys Val Phe Leu Asn Ile Gly Asp Lys Phe Leu Leu
                   85                  90                  95
   gac gcc aac ctg ggt aaa ggt gaa ggc gac aaa gaa aaa gtc ggt atc     336
   Asp Ala Asn Leu Gly Lys Gly Glu Gly Asp Lys Glu Lys Val Gly Ile
               100                 105                 110
   gac tac aaa ggc ctg cct gct gac gtc gtg cct ggt gac atc ctg ctg     384
   Asp Tyr Lys Gly Leu Pro Ala Asp Val Val Pro Gly Asp Ile Leu Leu
           115                 120                 125
   ctg gac gat ggt cgc gtc cag tta aaa gta ctg gaa gtt cag ggc atg     432
   Leu Asp Asp Gly Arg Val Gln Leu Lys Val Leu Glu Val Gln Gly Met
       130                 135                 140
   aaa gtg ttc acc gaa gtc acc gtc ggt ggt ccc ctc tcc aac aat aaa     480
   Lys Val Phe Thr Glu Val Thr Val Gly Gly Pro Leu Ser Asn Asn Lys
   145                 150                 155                 160
   ggt atc aac aaa ctt ggc ggc ggt ttg tcg gct gaa gcg ctg acc gaa     528
   Gly Ile Asn Lys Leu Gly Gly Gly Leu Ser Ala Glu Ala Leu Thr Glu
                   165                 170                 175
   aaa gac aaa gca gac att aag act gcg gcg ttg att ggc gta gat tac     576
   Lys Asp Lys Ala Asp Ile Lys Thr Ala Ala Leu Ile Gly Val Asp Tyr
               180                 185                 190
   ctg gct gtc tcc ttc cca cgc tgt ggc gaa gat ctg aac tat gcc cgt     624
   Leu Ala Val Ser Phe Pro Arg Cys Gly Glu Asp Leu Asn Tyr Ala Arg
           195                 200                 205
   cgc ctg gca cgc gat gca gga tgt gat gcg aaa att gtt gcc aag gtt     672
   Arg Leu Ala Arg Asp Ala Gly Cys Asp Ala Lys Ile Val Ala Lys Val
       210                 215                 220
   gaa cgt gcg gaa gcc gtt tgc agc cag gat gca atg gat gac atc atc     720
   Glu Arg Ala Glu Ala Val Cys Ser Gln Asp Ala Met Asp Asp Ile Ile
   225                 230                 235                 240
   ctc gcc tct gac gtg gta atg gtt gca cgt ggc gac ctc ggt gtg gaa     768
   Leu Ala Ser Asp Val Val Met Val Ala Arg Gly Asp Leu Gly Val Glu
                   245                 250                 255
   att ggc gac ccg gaa ctg gtc ggc att cag aaa gcg ttg atc cgt cgt     816
   Ile Gly Asp Pro Glu Leu Val Gly Ile Gln Lys Ala Leu Ile Arg Arg
               260                 265                 270
   gcg cgt cag cta aac cga gcg gta atc acg gcg acc cag atg atg gag     864
   Ala Arg Gln Leu Asn Arg Ala Val Ile Thr Ala Thr Gln Met Met Glu
           275                 280                 285
   tca atg att act aac ccg atg ccg acg cgt gca gaa gtc atg gac gta     912
   Ser Met Ile Thr Asn Pro Met Pro Thr Arg Ala Glu Val Met Asp Val
       290                 295                 300
   gca aac gcc gtt ctg gat ggt act gac gct gtg atg ctg tct gca gaa     960
   Ala Asn Ala Val Leu Asp Gly Thr Asp Ala Val Met Leu Ser Ala Glu
   305                 310                 315                 320
   act gcc gct ggg cag tat ccg tca gaa acc gtt gca gcc atg gcg cgc     1008
   Thr Ala Ala Gly Gln Tyr Pro Ser Glu Thr Val Ala Ala Met Ala Arg
                   325                 330                 335
   gtt tgc ctg ggt gcg gaa aaa atc ccg agc atc aac gtt tct aaa cac     1056
   Val Cys Leu Gly Ala Glu Lys Ile Pro Ser Ile Asn Val Ser Lys His
               340                 345                 350
   cgt ctg gac gtt cag ttc gac aat gtg gaa gaa gct att gcc atg tca     1104
   Arg Leu Asp Val Gln Phe Asp Asn Val Glu Glu Ala Ile Ala Met Ser
           355                 360                 365
   gca atg tac gca gct aac cac ctg aaa ggc gtt acg gcg atc atc acc     1152
   Ala Met Tyr Ala Ala Asn His Leu Lys Gly Val Thr Ala Ile Ile Thr
       370                 375                 380
   atg acc gaa tcg ggt cgt acc gcg ctg atg acc tcc cgt atc agc tct     1200
   Met Thr Glu Ser Gly Arg Thr Ala Leu Met Thr Ser Arg Ile Ser Ser
   385                 390                 395                 400
   ggt ctg cca att ttc gcc atg tcg cgc cat gaa cgt acg ctg aac ctg    1248
   Gly Leu Pro Ile Phe Ala Met Ser Arg His Glu Arg Thr Leu Asn Leu
                   405                 410                 415
   act gct ctc tat cgt ggc gtt acg ccg gtg cac ttt gat agc gct aat    1296
   Thr Ala Leu Tyr Arg Gly Val Thr Pro Val His Phe Asp Ser Ala Asn
               420                 425                 430
   gac ggc gta gca gct gcc agc gaa gcg gtt aat ctg ctg cgc gat aaa    1344
   Asp Gly Val Ala Ala Ala Ser Glu Ala Val Asn Leu Leu Arg Asp Lys
           435                 440                 445
   ggt tac ttg atg tct ggt gac ctg gtg att gtc acc cag ggc gac gtg    1392
   Gly Tyr Leu Met Ser Gly Asp Leu Val Ile Val Thr Gln Gly Asp Val
       450                 455                 460
   atg agt acc gtg ggt tct act aat acc acg cgt att tta acg gta gag    1440
   Met Ser Thr Val Gly Ser Thr Asn Thr Thr Arg Ile Leu Thr Val Glu
   465                 470                 475                 480
   taa                                                                1443
<210> 16
   <211> 480
   <212> PRT
   <213> Escherichia coli
<400> 16
   Met Ser Arg Arg Leu Arg Arg Thr Lys Ile Val Thr Thr Leu Gly Pro
    1               5                  10                  15
   Ala Thr Asp Arg Asp Asn Asn Leu Glu Lys Val Ile Ala Ala Gly Ala
               20                  25                  30
   Asn Val Val Arg Met Asn Phe Ser His Gly Ser Pro Glu Asp His Lys
           35                  40                  45
   Met Arg Ala Asp Lys Val Arg Glu Ile Ala Ala Lys Leu Gly Arg His
       50                  55                  60
   Val Ala Ile Leu Gly Asp Leu Gln Gly Pro Lys Ile Arg Val Ser Thr
   65                  70                  75                  80
   Phe Lys Glu Gly Lys Val Phe Leu Asn Ile Gly Asp Lys Phe Leu Leu
                   85                  90                  95
   Asp Ala Asn Leu Gly Lys Gly Glu Gly Asp Lys Glu Lys Val Gly Ile
               100                 105                 110
   Asp Tyr Lys Gly Leu Pro Ala Asp Val Val Pro Gly Asp Ile Leu Leu
           115                 120                 125
   Leu Asp Asp Gly Arg Val Gln Leu Lys Val Leu Glu Val Gln Gly Met
       130                 135                 140
   Lys Val Phe Thr Glu Val Thr Val Gly Gly Pro Leu Ser Asn Asn Lys
   145                 150                 155                 160
   Gly Ile Asn Lys Leu Gly Gly Gly Leu Ser Ala Glu Ala Leu Thr Glu
                   165                 170                 175
   Lys Asp Lys Ala Asp Ile Lys Thr Ala Ala Leu Ile Gly Val Asp Tyr
               180                 185                 190
   Leu Ala Val Ser Phe Pro Arg Cys Gly Glu Asp Leu Asn Tyr Ala Arg
           195                 200                 205
   Arg Leu Ala Arg Asp Ala Gly Cys Asp Ala Lys Ile Val Ala Lys Val
       210                 215                 220
   Glu Arg Ala Glu Ala Val Cys Ser Gln Asp Ala Met Asp Asp Ile Ile
   225                 230                 235                 240
   Leu Ala Ser Asp Val Val Met Val Ala Arg Gly Asp Leu Gly Val Glu
                   245                 250                 255
   Ile Gly Asp Pro Glu Leu Val Gly Ile Gln Lys Ala Leu Ile Arg Arg
               260                 265                 270
   Ala Arg Gln Leu Asn Arg Ala Val Ile Thr Ala Thr Gln Met Met Glu
           275                 280                 285
   Ser Met Ile Thr Asn Pro Met Pro Thr Arg Ala Glu Val Met Asp Val
       290                 295                 300
   Ala Asn Ala Val Leu Asp Gly Thr Asp Ala Val Met Leu Ser Ala Glu
   305                 310                 315                 320
   Thr Ala Ala Gly Gln Tyr Pro Ser Glu Thr Val Ala Ala Met Ala Arg
                   325                 330                 335
   Val Cys Leu Gly Ala Glu Lys Ile Pro Ser Ile Asn Val Ser Lys His
               340                 345                 350
   Arg Leu Asp Val Gln Phe Asp Asn Val Glu Glu Ala Ile Ala Met Ser
           355                 360                 365
   Ala Met Tyr Ala Ala Asn His Leu Lys Gly Val Thr Ala Ile Ile Thr
       370                 375                 380
   Met Thr Glu Ser Gly Arg Thr Ala Leu Met Thr Ser Arg Ile Ser Ser
   385                 390                 395                 400
   Gly Leu Pro Ile Phe Ala Met Ser Arg His Glu Arg Thr Leu Asn Leu
                   405                 410                 415
   Thr Ala Leu Tyr Arg Gly Val Thr Pro Val His Phe Asp Ser Ala Asn
               420                 425                 430
   Asp Gly Val Ala Ala Ala Ser Glu Ala Val Asn Leu Leu Arg Asp Lys
           435                 440                 445
   Gly Tyr Leu Met Ser Gly Asp Leu Val Ile Val Thr Gln Gly Asp Val
       450                 455                 460
   Met Ser Thr Val Gly Ser Thr Asn Thr Thr Arg Ile Leu Thr Val Glu
   465                 470                 475                 480
<210> 17
   <211> 33
   <212> DNA
   <213> Artificial Sequence
<220>
   <223> Description of Artificial Sequence: primer
<400> 17
   ataggatcca tgacaaagta tgcattagtc ggt 33
<210> 18
   <211> 33
   <212> DNA
   <213> Artificial Sequence
<220>
   <223> Description of Artificial Sequence: primer
<400> 18
   atagagctcg atttacagaa tgtgacctaa ggt 33
<210> 19
   <211> 36
   <212> DNA
   <213> Artificial Sequence
<220>
   <223> Description of Artificial Sequence: primer
<400> 19
   tacattggat ccatgattaa gaaaatcggt gtgttg 36
<210> 20
   <211> 34
   <212> DNA
   <213> Artificial Sequence
<220>
   <223> Description of Artificial Sequence: primer
<400> 20
   atcattgtcg acttaataca gttttttcgc gcag 34
<210> 21
   <211> 30
   <212> DNA
   <213> Artificial Sequence
<220>
   <223> Description of Artificial Sequence: primer
<400> 21
   ataggatcca tggtgagcga acgcagacgc 30
<210> 22
   <211> 30
   <212> DNA
   <213> Artificial Sequence
<220>
   <223> Description of Artificial Sequence: primer
<400> 22
   tttgagctct tacagaacgt cgatcgcgtt 30
<210> 23
   <211> 45
   <212> DNA
   <213> Artificial Sequence
<220>
   <223> Description of Artificial Sequence: primer
<400> 23
   aaaggatcca agcttaagga gaaattaaaa tgcgacatcc tttag 45
<210> 24
   <211> 51
   <212> DNA
   <213> Artificial Sequence
<220>
   <223> Description of Artificial Sequence: primer
<400> 24
   aaagagctcg aattcgtcga cagatcttta ttaagcctgt ttagccgctt c 51
<210> 25
   <211> 46
   <212> DNA
   <213> Artificial Sequence
<220>
   <223> Description of Artificial Sequence: synthetic promoter Pa3m
<400> 25
   aattcgtggt ttaccacatg aagtaagacg gtataatgta ccacag 46
<210> 26
   <211> 46
   <212> DNA
   <213> Artificial Sequence
<220>
   <223> Description of Artificial Sequence: synthetic promoter Pa3m
<400> 26
   gcaccaaatg gtgtacttca ttctgccata ttacatggtg tcctag 46
<210> 27
   <211> 36
   <212> DNA
   <213> Artificial Sequence
<220>
   <223> Description of Artificial Sequence: primer
<400> 27
   ttaggatcct tttattcact aacaaatagc tggtgg 36
<210> 28
   <211> 37
   <212> DNA
   <213> Artificial Sequence
<220>
   <223> Description of Artificial Sequence: primer
<400> 28
   ccgtctagaa atgaggtcca gttcatccag tttacga 37
<210> 29
   <211> 33
   <212> DNA
   <213> Artificial Sequence
<220>
   <223> Description of Artificial Sequence: primer
<400> 29
   actggatcca tgtccaaaat cgtaaaaatc atc 33
<210> 30
   <211> 28
   <212> DNA
   <213> Artificial Sequence
<220>
   <223> Description of Artificial Sequence: primer
<400> 30
   taagagctct tatgcctggc ctttgatc 28
<210> 31
   <211> 37
   <212> DNA
   <213> Artificial Sequence
<220>
   <223> Description of Artificial Sequence: primer
<400> 31
   aaaggatccg gaggattgct aatgaaaaac atcaatc 37
<210> 32
   <211> 29
   <212> DNA
   <213> Artificial Sequence
<220>
   <223> Description of Artificial Sequence: primer
<400> 32
   aaagagctca ttaaccgcgc cacgcttta 29
<210> 33
   <211> 30
   <212> DNA
   <213> Artificial Sequence
<220>
   <223> Description of Artificial Sequence: primer
<400> 33
   ataggatcca tgtctgtaat taagatgacc 30
<210> 34
   <211> 30
   <212> DNA
   <213> Artificial Sequence
<220>
   <223> Description of Artificial Sequence: primer
<400> 34
   atagagctct tacttcttag cgcgctcttc 30